# EUROPEAN PATENT APPLICATION

(11) **EP 4 368 185 A1**
(43) Date of publication of application: **15.05.2024**
(21) Application number: 22206891.8
(22) Date of filing: 11.11.2022
(51) Int. Cl.: A61K 31/519, A61K 31/5377, A61K 31/69, A61P 35/02, A61K 45/06

(54) **COMBINATION OF MENIN INHIBITOR(S) AND IMMUNOPROTEASE INHIBITOR(S) FOR TREATMENT OF LEUKEMIA**

(71) Applicant: Universitätsmedizin Greifswald, 17475 Greifswald (DE)
(72) Inventor: Heidel, Florian, 39120 Magdeburg (DE); Schnöder, Tina, 17489 Greifswald (DE); Perner, Florian, 17493 Greifswald (DE); Tubio Santamaria, Nuria, 17489 Greifswald (DE)
(74) Representative: Altmann Stößel Dick Patentanwälte PartG mbB

(57) **Abstract**

In a first aspect, the invention relates to a combination of at least one menin inhibitor with at least one immunoproteasome inhibitor for use as medicament, preferably for use in the treatment of leukemia. A second aspect of the invention is related to a pharmaceutical preparation comprising at least one menin inhibitor and at least one immunoproteasome inhibitor, optionally one or more pharmaceutically acceptable carrier(s) and optionally one or more pharmaceutically acceptable adjuvant(s).

## Description

In a first aspect, the invention relates to a combination of at least one menin inhibitor with at least one immunoproteasome inhibitor for use as medicament, preferably for use in the treatment of leukemia. A second aspect of the invention is related to a pharmaceutical preparation comprising at least one menin inhibitor and at least one immunoproteasome inhibitor, optionally one or more pharmaceutically acceptable carrier(s) and optionally one or more pharmaceutically acceptable adjuvant(s).

Adult acute myeloid leukemia (AML) is a type of cancer in which the bone marrow makes a large number of abnormal blood cells. Leukemia may affect red blood cells, white blood cells, and platelets. Ancute leukemia such as AML progresses rapidly, and is typically fatal within weeks or months if left untreated (https://www.cancer.gov/types/leukemia/patient/adult-aml-treatment-pdq#section/all). There are different subtypes of AML including AML harboring MLL-rearrangements (MLLr) and AML driven by NPM1-mutations. While AML with MLLr is only detected in up to 10% of human acute leukemias and is thus not a prominent AML, NPM1-driven leukemia is more prominent - NPM1 mutations represent the most common genetic lesion in adult AML with about one third of cases.

The proliferative capacity of leukemias is often mediated by oncogenic alterations in epigenetic and transcriptional cellular programs. Gene fusions, such as MLL fusion proteins, can initiate and maintain acute leukemia and are usually characterized by the presence of relatively few concomitant mutations. It is, for example, known that oncogenic fusions of the MML complexes drive the formation of leucemia (Krivtsov and Armstrong, 2007; Krivtsov et al., 2006).

Leukemias harboring translocations involving chromosome 11q23 are characterized by rearrangements of the Mixed-Lineage-Leukemia-gene (*MLL1*, *KMT2A*). Most frequently, MLL1 is fused to the C-terminal portion of AF4, AF9, ENL and AF10. Patients diagnosed with acute myeloid leukemia (AML) harboring MLL-rearrangements (MLLr) have a particularly poor prognosis compared to those showing other balanced translocations or those without cytogenetic aberrations. While the leukemic bulk can be eliminated in the majority of patients, the disease maintaining clone is not eliminated by chemotherapy, an effect that has been attributed to a persistent leukemia stem cell (LSC) pool that is inherently resistant to conventional cytotoxic agents.

NPM1-driven leukemia, which is more prominent than AML with MLLr in view of the number of subjects suffering from this disease, is based on the nucleophosmin (NPM1) gene, which encodes for a multifunctional protein with prominent nucleolar localization that shuttles between nucleus and cytoplasm.

It was already shown that inhibition of epigenetic proteins of the MML-complexes, such as Dot1L or menin, may reduce both, i.e. MLLr- and NPM1-driven leukemia formation, *in vitro* and in animal models (Bernt et al., 2011; Kuhn et al., 2016; Uckelmann et al., 2020). Pharmacological inhibition of Dot1L and menin resulted in clinical studies result in a relevant response of patients; however said response was limited and does not result in curing of the disease (Stein et al., 2021; Stein et al., 2018). While pharmacological inhibition of epigenetic proteins (e.g., Dot1L or menin) within the MLL complex has shown significant effects *in vitro,* initial clinical studies with these inhibitors *in vivo* have shown more transient responses and development of resistances.

Thus, a response of patients with MLLr orNPM1 driven leukemia towards a treatment with menin inhibitors is restricted and does, at least for the majority of the patients, not result in curing of the disease but rather leads to development of resistances or to recurrence.

It was already tried to combine a menin inhibitor based therapy with pharmacological inhibitors, which inhibit other cellular functions. For example, a combination of menin inhibitors with Bcl2 inhibitors such as Venetoclax [4-(4-{[2-(4-Chlorophenyl)-4,4-dimethyl-1-cyclohexen-1-yl]methyl}-1-piperazinyl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide] was investigated. Bcl-2 (B-cell lymphoma 2) is a protein, which is overexpressed in chronic lymphocytic leukemia (CLL), non-Hodgkin's lymphoma (NHL) and other B-cell lymphomas and which is involved in suppressing the natural process of programmed cell death (apoptosis). Blocking Bcl-2 restores the signaling cascade that causes cancer cells to destroy themselves. However, also such a combination of menin inhibitors and Bcl2 inhibitor does not give sufficient results, especially not in view of the much higher toxicity profile due to apoptosis.

An object of the present invention was thus the provision of a medicament, which shows significant effects not only *in vitro* but also *in vivo,* which is effective in treatment of leukemia and, quite important, which allows to avoid or at least reduce development of resistances.

### 1st aspect - combination of at least one menin inhibitor with at least one immunoproteasome inhibitor for use as medicament

According to a first aspect of the invention ,the problem was solved by combination of at least one menin inhibitor with at least one immunoproteasome inhibitor for use as medicament.

In some embodiments, the combination of at least one menin inhibitor with at least one immunoproteasome inhibitor is for use in treatment of leukemia.

As mentioned above, the proliferation capacity of leukemias is often caused by oncogenic changes of the epigenetic and transcriptional cellular programs. Gene fusions such as MLL fusion proteins may trigger and maintain an acute leukemia and are normally characterized by appearance of a relative small number of accompanying mutations. While the pharmacological inhibition of epigenetic proteins such as Dot1L or (MEN1) within the MLL complexes causes significant effects *in vitro,* first clinical studies with these inhibitors showed moreover a transient response and development of resistances. Most recently, mutations in menin could be identified *in vitro* and *in vivo* as the main reason for menin inhibitor resistance (unpublished data of Perner et al., to be published in 2022).

For identifying secondary dependencies of MLLr in acute myeloid leukemia, high-resolution proteome analyses were carried out. The functional dependency from catalytic immunoproteasome subunits was validated by CRISPR-Cas9 mediated deletion. Here, the proteasome unit PSMB8/LMP7 was identified as relevant target. Genetic and pharmacological deactivation of PSMB8/LMP7 resulted in a decrease of proliferation capacity, loss of self renewing capacity, reduction of oncogenic gene expression and displacement of MLL altering leukemia cells in bone marrow. On the contrary, the normal blood formation was not impaired by the inhibition of PSMB8/LMP7. Based on genetic screenings and the analysis of proteins, which are specifically dependent for their removal on PSMB8/LMP7, genes were identified that are relevant for leukemia. Overexpression of the regulator BASP1 resulted in an inhibition of proliferation and of MLLr dependent gene expression.

While pharmacological inhibition of PSMB8/LMP7 alone resulted in a delay and reduction of the development of MLLr mutated leukemia and NPM1 mutated leukemias *in vitro* and in animal model *in vivo,* no eradication or curing of the leukemias could be achieved. Here, the combination of menin inhibitors with pharmacological PSMB8 inhibition by immunoproteasome inhibitors gave an additive or synergistic effect. *In vitro,* a strong inhibition of proliferation was the result. In an animal model *in vivo,* the combinatory treatment resulted in a significantly increase in response and in the end in an eradication of the leukemia cells. Overall, the combination of menin inhibitors with a pharmacological inhibition of the immunoproteasome due to inhibitors of the PSMB8/LMP7 unit by immunoproteasome inhibitors is an innovative tool, which enables potentially the curing of patients with hardly treatable leukemias, especially leukemias with MLLr- and NPM1-mutations. Further details as well as preferred and/or alternative embodiments of the invention are described in the following.

In some embodiments of the combination for use as medicament, preferably for use in treatment of leukemia, the at least one menin inhibitor is a compound selected from the group consisting of MI-503, SNDX-5613, KO-539, MI-3454, mixtures of two or more of these menin inhibitors.

The term "menin inhibitor" comprises compounds as indicated above and pharmaceutically acceptable salts of these compounds. An "inhibitor" is a substance that influences one or more reactions in such a way that they are slowed down, inhibited or prevented. Inhibitors are used as enzyme-inhibiting substances. "Menin" is a protein that in humans is encoded by the *MEN1* gene and which participates in DNA repair and developmental processes in the nucleus of vertebrates and insects. Inter alia, as already indicated above, menin is also critical for leukemogenesis and thus for leukemias harboring translocations involving chromosome 1 1q23, which are characterized by rearrangements of the Mixed-Lineage-Leukemia-gene 1 (*MLL1, KMT2A; AML with MLLr*)*.* A "menin inhibitor" is a compound that is at least partially able to inhibit the effect of menin. Regarding the group of menin inhibitors indicated above, according to IUPAC nomenclature, MI-503 is 4-Methyl-1-(1H-pyrazol-4-ylmethyl)-5-[[4-[[6-(2,2,2-trifluoroethyl)thieno[2,3-d]pyrimidin-4-yl]amino]-1-piperidinyl]methyl]-1H-indole-2-carbonitrile; SNDX-5613 is N-ethyl-2-((4-(7-(((1r,4r)-4-(ethylsulfonamido)cyclohexyl)methyl)-2,7-diazaspiro[3.5]nonan-2-yl)pyrimidin-5-yl)oxy)-5-fluoro-N-isopropylbenzamide; KO-539 is (S)-4-Methyl-5-((4-((2-(methylamino)-6-(2,2,2-trifluoroethyl)thieno(2,3-d)pyrimidin-4-yl)amino)piperidin-1-yl)methyl)-1-(2-(4-(methylsulfonyl)piperazin-1-yl)propyl)-1H-indole-2-carbonitrile; and MI-3454 is N-(3-((2-cyano-4-methyl-5-((4-((2-(methylamino)-6-(2,2,2-trifluoroethyl)thieno[2,3-d]pyrimidin-4-yl)amino)piperidin-1-yl)methyl)-1H-indol-1-yl)methyl)bicyclo[1.1.1]pentan-1-yl)formamide. In some preferred embodiments of the combination for use as medicament, preferably for use in treatment of leukemia, the at least one menin inhibitor comprises at least MI-503.

In some preferred embodiments of the combination for use as medicament, preferably for use in treatment of leukemia, the at least one immunoproteasome inhibitor is a compound selected from the group consisting of Bortezomib, Carfilzomib, PR-957 (ONX-0914), M3258 and mixtures of two or more of these immunoproteasome inhibitors. In some preferred embodiments of the combination for use as medicament, preferably for use in treatment of leukemia, the at least one immunoproteasome inhibitor comprises at least PR-957.

The term "immunoproteasome inhibitor" comprises compounds as indicated above and pharmaceutically acceptable salts of these compounds. An "inhibitor", as defined above, is a substance that influences one or more reactions in such a way that they are slowed down, inhibited or prevented. Inhibitors are used as enzyme-inhibiting substances. Proteasomes are protein complexes which degrade unneeded or damaged proteins by proteolysis. An immunoproteasome is a type of proteasome that degrades ubiquitin-labeled proteins found in the cytoplasm in cells exposed to oxidative stress and proinflammatory stimuli. An "immunoproteasome inhibitor" is thus a compound that is able to at least partially inhibit the effect of an immunoproteasome. Regarding the group of immunoproteasome inhibitors indicated above, according to IUPAC nomenclature, Bortezomib is [(1R)-3-methyl-1-({(2S)-3-phenyl-2-[(pyrazin-2-ylcarbonyl)amino]propanoyl}-amino)butyl]boronic acid; Carfilzomib is (2S)-4-Methyl-N-[(2S)-1-[[(2S)-4-methyl-1-[(2R)-2-methyloxiran-2-yl]-1-oxopentan-2-yl]amino]-1-oxo-3-phenylpropan-2-yl]-2-[[(2S)-2-[(2-morpholin-4-ylacetyl)amino]-4-phenylbutanoyl]amino]pentanamide; PR-957 (ONX-0914) is (2S)-3-(4-methoxyphenyl)-N-[(2S)-1-[(2S)-2-methyloxiran-2-yl]-1-oxo-3-phenylpropan-2-yl]-2-[[(2S)-2-[(2-morpholin-4-ylacetyl)amino]propanoyl]amino]propanamide; and M3258 is ((S)-2-(benzofuran-3-yl)-1-((1R,2R,4S)-7-oxabicyclo[2.2.1]heptane-2-carboxamido)ethyl)boronic acid.

As used herein the term "pharmaceutically acceptable salts" refers to addition salts of the menin inhibitors and/or of immunoproteasome inhibitors with acids or bases that form anions or cations, which are, preferably, non -toxic to the recipient thereof. Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p -toluenesulfonate), trifluoroacetate, and undecanoate salts. Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N -methyl -D -glucamine; and salts with amino acids such as arginine, lysine, ornithine, and so forth. Also, basic nitrogen -containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others.

In some preferred embodiments of the combination for use as medicament, preferably for use in treatment of leukemia, the at least one menin inhibitor and the at least one immunoproteasome inhibitor are present in a molar ratio at least one menin inhibitor : at least one immunoproteasome inhibitor in the range of from 1:100 to 100:1, preferably in a molar ratio in the range of from 1:10 to 10:1.

In some preferred embodiments of the combination for use as medicament, preferably for use in treatment of leukemia, the at least menin inhibitor and the at least one immunoproteasome inhibitor are administered simultaneously consecutively or staggered, wherein in a staggered administration, preferably the administration of the at least one menin inhibitor starts prior to administration of the at least one immunoproteasome inhibitor.

In some preferred embodiments of the combination for use as medicament, preferably for use in treatment of leukemia, the at least one menin inhibitor is administered, preferably daily, for 1 to 14 consecutive days prior to start of the administration of the at least one immunoproteasome inhibitor.

In some preferred embodiments of the combination for use as medicament, preferably for use in treatment of leukemia, in simultaneous administration or in staggered administration after start of the administration of the at least one immunoproteasome inhibitor, the at least one menin inhibitor and the at least one immunoproteasome inhibitor are administrated, preferably both daily, together for a period of time of at least two days.

In some preferred embodiments of the combination for use as medicament, preferably for use in treatment of leukemia, n the combination is present in form of a pharmaceutical preparation.

In some preferred embodiments of the combination for use as medicament, preferably for use in treatment of leukemia, the pharmaceutical preparation comprises the at least one menin inhibitor and the at least one immunoproteasome inhibitor and one or more pharmaceutically acceptable carrier(s) and optionally one or more pharmaceutically acceptable adjuvant(s).

In some preferred embodiments of the combination for use as medicament, preferably for use in treatment of leukemia, the leukemia is selected from the group of acute myeloid leukemias (AML), and is preferably an AML associated with rearrangement(s) in the Mixed-Lineage-Leukemia gene 1 (MLL1-r-AML) or an AML associated with NPM1 mutation(s).

### 2^{nd} aspect - Pharmaceutical preparation

The invention is also related to a pharmaceutical preparation comprising at least one menin inhibitor and at least one immunoproteasome inhibitor, optionally one or more pharmaceutically acceptable carrier(s) and optionally one or more pharmaceutically acceptable adjuvant(s).

All details, preferred embodiments and alternative embodiments disclosed above in the section related to the first aspect of the invention apply also in the context of the second aspect, i.e. for the pharmaceutical composition.

The term "pharmaceutical composition", as used herein, relates to a mixture of compounds comprising at least the at least one menin inhibitor and/or the at least one immunoproteasome inhibitor or pharmaceutically acceptable salts thereof as specified herein and, preferably, at least one carrier. The at least one menin inhibitor and/or the at least one immunoproteasome inhibitor and/or a pharmaceutically acceptable salts thereof, comprised in the pharmaceutical composition are described herein above. The pharmaceutical composition may have any consistency deemed appropriate by the skilled person. In some preferred embodiments of the pharmaceutical preparation comprising at least one menin inhibitor and at least one immunoproteasome inhibitor, the dosage units comprise the at least one menin inhibitor and/or the at least one immunoproteasome inhibitor in liquid or solid form, preferably in a liquid form suitable for subcutaneous, intravenous (iv) or oral application or in a solid form suitable for oral application.

The pharmaceutical composition preferably comprises a carrier. The carrier(s) preferably is/are acceptable in the sense of being compatible with the other ingredients of the pharmaceutical composition and being not deleterious to a potential recipient thereof. The carrier(s) preferably is/are selected so as not to affect the biological activity of the pharmaceutical composition. Preferably, the pharmaceutical composition is sterile, more preferably a sterile solution, most preferably a sterile solution for injection. The carrier is selected by the skilled person such as to achieve the consistency intended and may be, for example, a gel or, preferably, a liquid, more preferably an aqueous liquid. Examples of such carriers are distilled water, physiological saline, Ringer's solutions, dextrose solution, phosphate -buffered saline solution, and Hank's solution. The carrier may include one or more solvents or other ingredients increasing solubility of the compounds comprised in the pharmaceutical composition. For example, Bortezomib may be used, for instance if intended for iv injection, dissolved in physiological saline, Carfilzomib may be used in dextrose solution and PR-957 may be dissolved in physiological saline as well. Further examples of liquid carriers are syrup, oil such as peanut oil and olive oil, water, emulsions, various types of wetting agents, sterile solutions and the like. Suitable carriers comprise those mentioned above and others well known in the art. As is understood by the skilled person, the pharmaceutical composition may comprise one or more further compounds; preferably, such additional compounds are selected so as to not affect the biological activity of the pharmaceutical composition, in particular of the active the at least one menin inhibitor and/or the at least one immunoproteasome inhibitor and/or a pharmaceutically acceptable salts thereof, is acceptable in the sense of being compatible with the other ingredients of the pharmaceutical composition and being not deleterious to a potential recipient thereof.

Preferably, the carrier is a pharmaceutically acceptable carrier. In addition, the pharmaceutical composition or formulation may also include other carriers, adjuvants, stabilizers and/or other compounds deemed appropriate by the skilled person, e.g. for galenic purposes. As referred to herein, the at least one menin inhibitor and/or the at least one immunoproteasome inhibitor or pharmaceutically acceptable salts thereof as specified, is/are the "active compound" of the preparation, although "further active compounds", which are referred to under this term, may be present. Specific pharmaceutical compositions are prepared in a manner well known in the pharmaceutical art and comprise at least one active compound referred to herein above, preferably in admixture or otherwise associated with at least one pharmaceutically acceptable carrier or diluent. For making those specific pharmaceutical compositions, the active compound(s) will usually be mixed with a carrier or the diluent. The resulting formulations are to be adapted to the mode of administration, i.e. in the forms of tablets, capsules, suppositories, solutions, suspensions or the like.

Dosage recommendations shall be indicated in the prescriber's or user's instructions in order to anticipate dose adjustments depending on the considered recipient.

The pharmaceutical composition is, preferably, administered in conventional dosage forms prepared by combining the active compound with standard pharmaceutical carriers according to conventional procedures. These procedures may involve mixing or dissolving the ingredients as appropriate to obtain the desired preparation. It will be appreciated that the form and character of the pharmaceutically acceptable carrier or diluent is dictated by the amount of active ingredient with which it is to be combined, the route of administration and other well -known variables. Similarly, the carrier or diluent may include time delay material well known in the art, such as glyceryl mono -stearate or glyceryl distearate alone or with a wax. A therapeutically effective dose refers to an amount of the active compound to be used in a pharmaceutical composition of the present invention which provides the effect referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors; preferably in accordance with any one of the above described methods. As is well known in the medical arts, dosages for any one patient depend upon many factors, which may include the patient's size, body surface area, age, the particular compound to be administered, sex, time and route of administration, general health, and other drugs being administered concurrently. Progress can be monitored by periodic assessment. A typical dose can be, for example, in the range of 1 µg to 1000 mg; however, doses below or above this exemplary range are envisioned, especially considering the aforementioned factors. Generally, the regimen as a regular administration of the pharmaceutical composition should be in the range of 1 µg to 100 mg units per day. If the regimen is a continuous infusion, it should also be in the range of 1 µg to 1 mg units per kilogram of body weight per minute, respectively. Depending on the subject and the mode of administration, the quantity of substance administration may vary over a wide range to provide from about 0.01 mg per kg body mass to about 100 mg per kg body mass. The pharmaceutical compositions and formulations referred to herein are administered preferably administered more than one time, more preferably on a continuous basis and/or in regular intervals. Preferably, administration is adjusted to maintain an effective concentration in the body of a subject for the time period intended.

In some preferred embodiments of the pharmaceutical preparation comprising at least one menin inhibitor and at least one immunoproteasome inhibitor, the at least one menin inhibitor and at least one immunoproteasome inhibitor are present in the same dosage unit or are present in separated dosage units.

In some preferred embodiments of the pharmaceutical preparation comprising at least one menin inhibitor and at least one immunoproteasome inhibitor, the pharmaceutical preparation comprises one or more dosage units, wherein the at least one menin inhibitor and at least one immunoproteasome inhibitor are present together in each dosage unit, optionally combined with one or more dosage units comprising the at least one menin inhibitor as the sole active ingredient (i.e. without the at least one immunoproteasome inhibitor).

In some preferred embodiments of the pharmaceutical preparation comprising at least one menin inhibitor and at least one immunoproteasome inhibitor, the pharmaceutical preparation comprises one or more dosage units comprising the at least one menin inhibitor as the sole active ingredient and one or more dosage units comprising the at least one immunoproteasome inhibitor as the sole active ingredient, wherein optionally, the sum of dosage units comprising the at least one menin inhibitor as the sole active ingredient is larger than the sum of dosage units comprising the at least one immunoproteasome inhibitor as the sole active ingredient. "As the sole active ingredient" means that the respective inhibitor is prominently present, whereas the other inhibitor is only present in amounts too low to have an effect when administered. Preferably, the other inhibitor is present in amounts ≤ 1 % by weight, more preferably ≤ 0.1 % by weight, more preferably ≤ 0.01 % by weight, based on the total weight of the dosage unit being 100 % by weight. For example, in a dosage unit having the at least one menin inhibitor as the sole active ingredient, the at least one immunoproteasome inhibitor is present in amounts ≤ 1 % by weight, more preferably ≤ 0.1 % by weight, more preferably ≤ 0.01 % by weight, based on the total weight of the dosage unit being 100 % by weight.

In some embodiments, the invention is also directed to a method for treating a leukemia, preferably acute myeloid leukemias (AML), more preferably an AML associated with rearrangement(s) in the Mixed-Lineage-Leukemia gene 1 (MLL1-r-AML) or an AML associated with NPM1 mutation(s), in a subject suffering therefrom, comprising
(a) administering at least one menin inhibitor and at least one immunoproteasome inhibitor to said subject, and
(b) thereby treating said leukemia.

In some embodiments the invention relates to a method for treating a leukemia comprising administering a pharmaceutical composition such as described above in the section related to the second aspect. All details, preferred embodiments and alternative embodiments disclosed above in the section related to the first aspect of the invention also apply here for the method of treatment.

The method for treating, preferably, is an *in vivo* method. Moreover, it may comprise steps in addition to those explicitly mentioned above. For example, further steps may relate, e.g., to diagnosing leukemia and/or identifying the subject as profiting from treatment with a combination as described above; thus, preferably, the method comprises the further steps of (i) determining or having determined leukemia in a subject; and (ii) identifying or having identified a subject profiting from treatment with a combination as described above. Moreover, one or more of said steps may be performed by automated equipment.

The present invention is further illustrated by the following set of embodiments and combinations of embodiments resulting from the dependencies and back-references as indicated. In particular, it was noted that in each instance where a range of embodiments was mentioned, for example in the context of a term such as " any one of embodiments (1) to (4)", every embodiment in this range was meant to be explicitly disclosed for the skilled person, i.e. the wording of this term was to be understood by the skilled person as being synonymous to "any one of embodiments (1), (2), (3) and (4)". Further, it was explicitly noted that the following set of embodiments was not the set of claims determining the extent of protection, but represents a suitably structured part of the description directed to general and preferred aspects of the present invention.
1. Combination of at least one menin inhibitor with at least one immunoproteasome inhibitor for use as medicament.
2. Combination of at least one menin inhibitor with at least one immunoproteasome inhibitor for use in treatment of leukemia.
3. Combination for use according to embodiment 1 or 2, wherein the at least one menin inhibitor is a compound selected from the group consisting of MI-503, SNDX-5613, KO-539, MI-3454, mixtures of two or more of these menin inhibitors.
4. Combination for use according to embodiment 3, wherein the at least one menin inhibitor comprises at least MI-503.
5. Combination for use according to any one of embodiments 1 to 4, wherein the at least one immunoproteasome inhibitor is a compound selected from the group consisting of Bortezomib, Carfilzomib, PR-957 (ONX-0914), M3258 and mixtures of two or more of these immunoproteasome inhibitors.
6. Combination for use according to embodiment 5, wherein the at least one immunoproteasome inhibitor comprises at least PR-957.
7. Combination for use according to any one of embodiments 1 to 6, wherein the at least one menin inhibitor and the at least one immunoproteasome inhibitor are present in a molar ratio at least one menin inhibitor : at least one immunoproteasome inhibitor in the range of from 1:100 to 100:1, preferably in a molar ratio in the range of from 1:10 to 10:1.
8. Combination for use according to any one of embodiments 1 to 7, wherein the at least menin inhibitor and the at least one immunoproteasome inhibitor are administered simultaneously consecutively or staggered, wherein in a staggered administration, preferably the administration of the at least one menin inhibitor starts prior to administration of the at least one immunoproteasome inhibitor.
9. Combination for use according to embodiment 8, wherein the at least one menin inhibitor is administered, preferably daily, for 1 to 14 consecutive days prior to start of the administration of the at least one immunoproteasome inhibitor.
10. Combination for use according to embodiment 8 or 9, wherein in simultaneous administration or in staggered administration after start of the administration of the at least one immunoproteasome inhibitor, the at least one menin inhibitor and the at least one immunoproteasome inhibitor are administrated, preferably both daily, together for a period of time of at least two days.
11. Combination for use according to any one of embodiments 1 to 10, wherein the combination is present in form of a pharmaceutical preparation.
12. Combination for use according to embodiment 11, wherein the pharmaceutical preparation comprises the at least one menin inhibitor and the at least one immunoproteasome inhibitor and one or more pharmaceutically acceptable carrier(s) and optionally one or more pharmaceutically acceptable adjuvant(s).
13. Combination for use according to any one of embodiments 1 to 12, wherein the leukemia is selected from the group of acute myeloid leukemias (AML), and is preferably an AML associated with rearrangement(s) in the Mixed-Lineage-Leukemia gene 1 (MLL1-r-AML) or an AML associated with NPM1 mutation(s).
14. Pharmaceutical preparation comprising at least one menin inhibitor and at least one immunoproteasome inhibitor, optionally one or more pharmaceutically acceptable carrier(s) and optionally one or more pharmaceutically acceptable adjuvant(s).
15. Pharmaceutical preparation comprising at least one menin inhibitor and at least one immunoproteasome inhibitor according to embodiment 14, wherein the at least one menin inhibitor and at least one immunoproteasome inhibitor are present in the same dosage unit or are present in separated dosage units.
16. Pharmaceutical preparation comprising at least one menin inhibitor and at least one immunoproteasome inhibitor according to embodiment 14 or 15, comprising one or more dosage units, wherein the at least one menin inhibitor and at least one immunoproteasome inhibitor are present together in each dosage unit, optionally combined with one or more dosage units comprising the at least one menin inhibitor as the sole active ingredient (i.e. without the at least one immunoproteasome inhibitor).
17. Pharmaceutical preparation comprising at least one menin inhibitor and at least one immunoproteasome inhibitor according to embodiment 14 or 15, comprising one or more dosage units comprising the at least one menin inhibitor as the sole active ingredient (i.e. without the at least one immunoproteasome inhibitor) and one or more dosage units comprising the at least one immunoproteasome inhibitor as the sole active ingredient (i.e. without the at least one menin inhibitor), wherein optionally, the sum of dosage units comprising the at least one menin inhibitor as the sole active ingredient is larger than the sum of dosage units comprising the at least one immunoproteasome inhibitor as the sole active ingredient.
18. Pharmaceutical preparation comprising at least one menin inhibitor and at least one immunoproteasome inhibitor according to any one of embodiments 14 to 17, wherein the dosage units comprise the at least one menin inhibitor and/or the at least one immunoproteasome inhibitor in liquid or solid form, preferably in a form suitable for subcutaneous, intravenous or oral application.

### EXAMPLES

### 1 Materials and Methods

### 1.1 Cell Lines

MOLM-13, MONO-MAC-6, THP-1, KOPN-8, ML-2, MV-4;11, OCI-AML3, U-937, KASUMI-1, K-562, HL-60 and LAMA-84 cells were purchased from DSMZ (Braunschweig, Germany). Cas9- Blast cell lines are subclones of the respective cell lines and stably transduced with lentiCas9-Blast (Addgene plasmid #52962). Cells were cultured in RPMI 1640 medium (Thermo Fisher Scientific, Waltham, MA, USA) supplemented with 10-20% FBS (Thermo Fisher Scientific) in a humid atmosphere of 5% CO2 at 37°C. MV4;11 cells harboring Menin-inhibitor resistance mediating mutations of Menin were generated by CRISPR-Cas9 technology.

### 1.2 Animal Studies

All mice were housed under pathogen-free conditions in the Animal Research Facility of the Otto-von-Guericke University Medical Faculty, Magdeburg and the ZET at the University Hospital Jena. All experiments were conducted after approval by the Landesverwaltungsamt Sachsen-Anhalt (42502-2-1279 UniMD) and Thüringen (02-030/2016). Mice harboring a constitutive knockout allele of LMP7-/- (Fehling et al., 1994) have been purchased from JAX.

### 1.3 Primary Patient Samples

Primary AML patient samples and healthy donor controls were obtained after informed consent and according to the Helsinki declaration within the AML-trials of the German-Austrian AMLSG study group and from the Hematology Tumor Bank Jena and Magdeburg, approved by the respective local ethics committee (Ethics Committee of the Medical Faculty, University Hospital Jena #4753/04-16 or University Hospital Magdeburg #115/08). Human bone marrow aspirates were separated with Ficoll-Paque Plus (GE Healthcare, Chicago, IL, USA) following the manufacturer's instruction and cryopreserved in 1x freezing medium (80% FBS + 10% DMSO + 10% IMDM medium).

### 1.4 Bone Marrow Transplantation Experiments and Drug Treatment

For induction of MLL-AF9 (MA9) driven leukemia we used established retroviral MSCV-GFP-based vectors to express MA9 in hematopoietic stem- and progenitor cells (Lineage-negative, Sca1-positive, Kit-positive [LSK] cells). In detail, bone marrow (BM) of LMP7+/+ or LMP7-/- littermates was isolated following standard procedures from 6-12-weeks-old mice. Lineage-depletion was performed using Streptavidin Particles Plus-DM (BD Biosciences, Heidelberg, Germany) and a DynaMag magnet (Thermo Fisher Scientific). Lineage-depleted cells were stained with streptavidin BV421, KIT AF647 and Sca-1 FITC or PE antibodies (all antibodies were received from Biolegend, San Diego, CA, USA) and then used for cell sorting. Sorted LSK cells were pre-stimulated on RetroNectin-coated plates (Takara Bio USA, Inc., Mountain View, CA, USA) in serum-free medium (StemSpan^{™}SFEM, StemCell Technologies, Vancouver, Canada) supplemented with SCF, TPO, IL-3 and IL-6 (all cytokines from PeproTech, Hamburg, Germany) overnight. 20 hours after stimulation, LSK cells were infected twice (8 hours gap) by spinfection (872g, 1.5 hours, 33°C) with retroviral particles containing the oncogenic vectors. Cells were analyzed 48 hours after first infection for GFP-expression using flow cytometry and 7.5×10⁴ GFP+ cells were injected into sublethally irradiated (7Gy, single dose) primary recipient mice (C57BL/6J; females). For serial transplantation of leukemia, 3×10⁵ whole bone marrow cells of diseased animals were transplanted into secondary C57BL/6J recipient mice (sublethal irradiation, 7Gy, single dose).. For competitive repopulation assays of normal hematopoiesis, 2×10⁶ BM cells of 6-8-weeks old B6.SJL-*Ptprc^{a}Pepc^{b}*/*BoyCrl* (CD45.1) and 2×10⁶ (CD45.1/2) competitor cells (derived from intercrossing B6.SJL-*Ptprc^{a}Pepc^{b}*/BoyCrl (purchased from Charles River) with C57BL/6JRj (CD45.2) animals (purchased from Janvier Labs)) were transplanted via lateral tail vein injection into lethally irradiated (13 Gy, single-dose) 6-8-weeks old C57BL/6JRj (CD45.2) recipient mice (females). PR- 957 was solved in DMSO and diluted in CAPTISOL^{®} from a sterile stock solution and administered by i.v. injections (3mg/KG, 6mg/KG or 10mg/KG as indicated) once daily as published before (Muchamuel et al., 2009). MI-503 was solved in 25% DMSO + 25% PEG-400 + 50% NaCl or solved in a diluted solution of DMSO in CAPTISOL^{®} and administered by i.p. injections at 50mg/KG. NaCl 0.9% or 30% DMSO-70% NaCl was injected as a negative control.

### 1.5 Mouse Xenotransplantation

NOD.Cg-Prkdc^{scid} Il2rg^{tm1Wjl} Tg(CMV-IL3, CSF2, KITLG) 1Eav/MloySzJ (NSGS), NOD.Cg-Prkdc^{scid} Il2rg^{tm1Wjl}/SzJ (NSG) or NOD.Cg-Prkdc^{scid} Il2rg^{tm1Wjl}/RJ (NXG) were obtained from The Jackson Laboratory (Bar Harbor, ME, USA). 8-12-weeks old adult mice (male and female) were irradiated with 2 Gy (single dose) before transplantation. 1×10⁵ MOLM-13, ML-2 or MV-4; 11 cells transduced with either of two PSMB8/LMP7 shRNAs or non-targeting control; with a pLEX vector containing the sequence of BASP1 or an empty vector; or treated *ex vivo* with the indicated inhibitors were injected intravenously via the tail vein. For patient derived xenograft experiments (PDX) of human MLLr leukemia we used an improved model for human HSC transplantation and analysis, that has been developed from immune-deficient mouse strains containing Kit mutations: NOD.Cg- KitW-41J Prkdc^{scid} Il2rg^{tm1Wjl}/WaskJ (NSGW41) (Cosgun et al., 2014). These mice can be engrafted without prior conditioning and therefore maintain an intact niche and microenvironment. Human myeloid engraftment (hCD45+ CD13+) was analyzed by flow cytometry.

### 1.6 Methylcellulose Colony-Forming Assays

Primary mouse bone marrow (BM) cells (transduced with MLL-AF9/KRAS, MLL-AF6, or AML1-ETO/KRAS) were seeded in MethoCult GF M3434 (StemCell Technologies) at a concentration of 1×10³ cells/replicate. Colonies propagated in culture were scored at day 8. For re-plating of primary mouse BM cells, colonies were harvested from the methylcellulose medium, washed with PBS/1%FBS and seeded again at a concentration of 1×10³ cells/replicate. MOLM-13 or ML-2 cells infected with either control or PSMB8/LMP7-specific shRNAs were plated at a concentration of 1×10³ cells in MethoCult H4230 (StemCell Technologies) supplemented with 20% FBS and colony numbers were counted on day 10-14.

### 1.7 Giemsa Staining and Immunohistochemistry

To analyze cell morphology, 1×10⁵ cells were centrifuged onto glass slides and stained with May-Grünwald/Giemsa staining as described before (Schnoder et al., 2015).

### 1.8 Flow Cytometry and Antibody Staining

Immunophenotyping of immature and mature cell compartments and of leukemic cells was performed as described before (Heidel et al., 2013; Heidel et al., 2012). The antibodies used for cell surface staining are provided in Table 1 at the end of section 2 ("Key Resource Table"). Cells were stained in PBS/1% FBS for 1.5 hours at 4°C. SYTOX^{®}Blue Dead Cell Stain (Life Technologies, Darmstadt, Germany) was used to exclude dead cells. Flow cytometry was performed on a LSR-Fortessa or FACS Canto II cytometer (Becton Dickinson, Heidelberg, Germany). Cell cycle analysis was performed using the Click-iT^{™} Plus EdU Alexa Fluor^{™} 647 Assay Kit (Thermo Scientific, Waltham, MA, USA) as per the manufacturer s instructions.

### 1.9 Genetic Inactivation by RNAi

For shRNA knockdown experiments, the mirN-vector system with puromycin resistance gene was used containing different shRNA sequences for PSMB8/LMP7. HEK293T cells were transfected using FUGENE^{®}HD Transfection Reagent (Promega, Madison, WI, USA) to generate lentiviral particles as described before (Schnoder et al., 2015). Target cells were infected twice (8 hours gap in between) by spinfection (872xg for 1.5 hours, at 33°C). 48 hours post-infection, puromycin was added in a concentration of 1 µg/ml for selection. The knockdown efficiency was assessed by quantitative real-time PCR (RT-qPCR) or Western Blotting 5-7 days post-infection. shRNA sequences are provided in Table 1 at the end of section 2 ("Key Resource Table").

### 1.10 Competitive CRISPR/Cas9 assay and Genome-wide CRISPR/Cas9 Screening

Guide RNAs targeting catalytic proteasome and immunoproteasome subunits were designed using the Broad GPP tool (https://portals.broadinstitute.org/gpp/public/analysis-tools/sgrna-design) (Doench et al., 2014) (sgRNA sequences are provided in Table 1 at the end of section 2 ("Key Resource Table"). For cloning sgRNA sequences, the improved-scaffold-pU6-sgRNA-EF1Alpha-PURO-T2A-RFP (ipUSEPR) vector system (Uckelmann *et al.,* 2020), with puromycin resistance and RFP selection marker was used. HEK293T cells were transfected using FUGENE^{®}HD Transfection Reagent (Promega, Madison, WI, USA) to generate lentiviral particles as described before (Schnoder *et al.,* 2015). Cell lines stably expressing Cas9 were infected twice (8 hours gap in between) by spinfection (872xg, 1.5 hours, 33°C). Two days post-infection, infected cells were mixed with non-infected Cas9 expressing cells to obtain a RFP+/RFP- ratio of 5-15%. RFP+/RFP-ratio was tracked every three days for a total of 12 days as described previously (Kühn et al., 2016; Shi et al., 2015) by flow cytometry. The cells expressing sgRNAs were selected with 1 µg/ml puromycin starting on day 2 post-infection. Cells were collected for RT-qPCR or Western Blotting on day 7 post-infection. The human lentiviral CRISPR/Cas9 library was developed and kindly provided by Dr. X. S. Liu (Addgene #1000000132). For the PR- 957 screen the H2 library, containing 92817 guide RNAs targeting against 18436 genes was used. Guide-RNAs were designed using an improved algorithm (Xu et al., 2015) and cloned into the lentiCRISPRv2 vector backbone (Addgene: #52961). The plasmid-library was electroporated into competent *E. coli Stbl4* and sequenced after Maxiprep to ensure a near-complete representation of guide-RNAs and homogeneous distribution of single guides (Gini-index at 0.1). For the *in vitro* screen, lentivirus was produced using HEK-293T cells transfected with the pooled library as well as the respective packaging plasmids (3ug pooled library, 3ug pMD2.G, 3ug psPAX2) using FuGENE according to the manufacturer's instructions (Heidel *et al.,* 2013). Lentivirus was harvested 48h and 58h after transfection, filtered (0.45um), pooled, snap-frozen and stored at -80C. To ensure a low MOI (1 single guide per gene in >90% of cells), the lentivirus was titrated on MOLM-13 cells to a transduction efficiency of 20-25%. To transduce the genome-scale CRISPR/Cas9 library at a proper representation into MOLM-13 cells, 6× 10⁸ cells were spinfected (872 g, 37C, 2h) in 6-well plates containing 4ml virus-containing cell culture medium and 1× 10⁷ cells per well. After the spin cells were transferred to T175 cell culture flasks and the virus-containing medium was diluted 1:1 with fresh complete medium (RPMI+10% FBS). After overnight incubation cells were washed from the viral particles and cultured in complete medium in T175 flasks at a density of 1× 10⁶/ml for 2 days. Subsequently, puromycin was added at a concentration of 1.5ug/ml and cells were selected for 4 days. After selection of guide-expressing cells the percentage of dead cells as well as the total number of living cells were determined by flow cytometry. To maintain representation, 4x 10⁷ cells per replicate have been assigned to the PR-957 treatment or diluent control (DMSO) groups of the screen (4 replicates per group). 5x 10⁷ cells were purified from apoptotic cells using the AnnexinV-based EasySep^{™} Dead Cell Removal Kit (Stem Cell Technologies) and snap-frozen as a pellet (baseline-sample). Cells in the PR-957-arm of the screen were treated with increasing concentrations of the drug (50-200nM) until a clear decline in total viable cell numbers was observed and the IC50 was reached. During the screen, cells have been splitted to contain a minimum number of 4× 10⁷ viable cells in every single replicate. The cell density was constantly maintained between 0.5 and 1.5× 10⁶ cells per ml. On day 12 after start of PR-957 treatment cells were harvested and apoptotic cells were removed. Cell pellets of 5× 10⁷ viable cells were snap-frozen and stored at -80C. Genomic DNA was isolated from these pellets using phenol-chloroform extraction and subsequently eluted in TE buffer. Concentration was determined by Nanodrop. Library preparation was performed according to standard protocols (https://www. addgene.org/pooled-library/liu-crispr-knockout/).

### 1.11 Quantitative Real-Time PCR

Equal amounts (1 µg) of total RNA were extracted using TRIzol Regent (Thermo Fisher Scientific, Waltham, MA, USA) or the RNeasy Mini Kit (Qiagen, Hilden, Germany). RNA was reverse-transcribed using Omniscript RT Kit (Qiagen, Hilden, Germany) as per the manufacturer's instructions and complementary DNA samples were analyzed by RT-qPCR using SYBR^{®} Premix Ex TaqII^{™} (Clontech Laboratories, Mountain View, CA, USA) following the manufacturer's instruction. Gene-specific primers were designed to span exon-exon boundaries to avoid amplification of genomic DNA. RT-qPCR data were normalized to beta-2-microglobulin. All expression values were normalized and standardized using the Bio-Rad CFX Manager software (Munich, Germany) and presented as fold changes of gene expression in the test sample compared to the control. Primer sequences are listed in Table 1 at the end of section 2 ("Key Resource Table").

### 1.12 Cellular Proliferation & Apoptosis

Cellular proliferation was assessed by cell counting after trypan blue exclusion. Apoptosis of cells was measured by flow cytometry using Annexin V/ SYTOX^{®}Blue staining following the manufacturer's instruction.

### 1.13 Protein Extraction and Immunoblotting

Cells were washed twice with ice-cold PBS and lysed in lysis buffer (50 mM HEPES pH7.4, Glycerol 10%, NaCl 150 mM, TritonX-100 1%, MgCl 1.5 mM, EGTA 5mM) for 1 hour on ice. Protein concentration was calculated using the Protein Assay Dye Reagent Concentrate (Bio-Rad Laboratories, Inc., Hercules, CA, USA) following the manufacturer's instruction. Antibodies are listed in Table 1 at the end of section 2 ("Key Resource Table").

### 1.14 Global Proteome Analysis

For global proteome profiling, leukemia development was initiated with MLL-AF9 (or AML1-ETO as control) containing MSCV-GFP constructs. Murine stem-and progenitor cells (LSK cells: Lin-Sca+Kit+) from 6-8 weeks-old C57BL/6J donors (females) were sorted and infected by co- localization of virus supernatant (containing one of the oncogenes) with LSK cells on retronectin-coated plates. 72 hours after infection equal numbers of GFP+ cells were injected into sublethally irradiated recipient hosts (7Gy). 2× 10⁵ LSC-enriched (GFP+Kit+) cells (4 replicates per oncogene) were sorted directly into 2x lysis buffer (for a final concentration: 1% SDS, 50 mM HEPES, pH 8.5, 10 mM DTT; volume of lysis buffer added to collection tube was estimated to be equal to the volume of the sheath buffer). Samples were sonicated in a Bioruptor (Diagenode, Belgium) (10 cycles with 1 minute on and 30s off with high intensity at 20°C). Samples were then heated at 95°C for 10 minutes, before being subjected to another round of sonication. The lysates were cleared, and debris precipitated by centrifugation (14,000 rpm for 10 minutes), then incubated with 15 mM iodacet-amide at room temperature 20 minutes. Each sample was treated with 8 volumes ice cold acetone and left overnight at -20°C to precipitate the proteins. The samples were then centrifuged at 14,000 rpm for 30 minutes at 4°C. After removal of the supernatant, the precipitates were washed twice with 500 µL of ice cold 80 % acetone. The pellets were then allowed to air-dry before being dissolved in digestion buffer (3M urea in 0.1 M HEPES, pH 8). To facilitate the resuspension of the protein pellet, the samples were subjected to 3 rounds of sonication in the Bioruptor, as described above. A 1:100 w/w amount of LysC (Wako sequencing grade) was added to each sample and samples were incubated for 4 h at 37°C on a shaker. The samples were diluted 1:1 with milliQ water (to reach 1.5M urea) and were incubated with a 1:100 w/w amount of trypsin (Promega, Madison, WI, USA) overnight at 37°C and 650 rpm. The digests were then acidified with 10% trifluoroacetic acid and desalted with *Waters Oasis^{®} HLB µElution Plate 30µm* in the presence of a slow vacuum. In this process, the columns were conditioned with 3 × 100 µL solvent B (80% acetonitrile; 0.05% formic acid) and equilibrated with 3x100 µL solvent A (0.05% formic acid in milliQ water). The samples were washed 3 times with 100 µL solvent A, and then eluted into PCR tubes with 50 µL solvent B. The eluates were dried down with the speed vacuum centrifuge and dissolved in 5% acetonitrile with 0.1% formic acid to a final volume of 10 µL, which was transferred to an MS vial and 0.25 µL of HRM kit peptides (Biognosys, Zurich, Switzerland) was spiked into each sample prior to analysis by LC-MS/MS. For the sample pools, 2 µL of each sample contained within the pool was removed from the individual samples and 1.5 µL of this was injected for each of the DDA runs, in triplicate. 4.5 µL was injected per individual sample for DIA acquisition. Peptides were separated using the nanoAcquity UPLC MClass system (Waters) fitted with a trapping (nanoAcquity Symmetry C18, 5µm, 180 µm × 20 mm) and an analytical column (nanoAcquity BEH C18, 1.7µm, 75µm × 250mm). The outlet of the analytical column was coupled directly to Q-Exactive HFX (Thermo Fisher Scientific) using the Proxeon nanospray source. Solvent A was water, 0.1 % formic acid and solvent B was acetonitrile, 0.1 % formic acid. The samples (either a subset of the samples (mouse) or a pool of samples, injected in triplicate) were loaded with a constant flow of solvent A at 5 µL/min onto the trapping column. Trapping time was 6 min. Peptides were eluted via the analytical column with a constant flow of 0.3 µL/min. During the elution step, the percentage of solvent B increased in a non-linear fashion from 0 % to 40 % in 60 min. Total runtime was 75 min, including clean-up and column re-equilibration. The peptides were introduced into the mass spectrometer via a Pico-Tip Emitter 360 µm OD × 20 µm ID; 10 µm tip (New Objective) and a spray voltage of 2.2 kV was applied. The capillary temperature was set at 300 °C. The RF ion funnel was set to 40%. Full scan MS spectra with mass range 350-1650 *m*/*z* were acquired in profile mode in the Orbitrap with resolution of 60000. The filling time was set at maximum of 20ms with an AGC target of 1×10⁶ ions. The peptide match algorithm was set to "preferred" and only charge states from 2⁺ to 5⁺ were selected for fragmentation. The top 15 most intense ions from the full scan MS were selected for MS2, using quadrupole isolation and a window of 1.6 Da. An intensity threshold of 4 ×10⁴ ions was applied. HCD was performed with normalized collision energy of 31%. A maximum fill time of 25ms, with an AGC target of 2×10⁵ for each precursor ion was set. MS2 data were acquired in profile, with a resolution of 15000 with fixed first mass of 120 *m*/*z.* The dynamic exclusion list was with a maximum retention period of 30 sec and relative mass window of 10 ppm. Isotopes were also excluded. In order to improve the mass accuracy, internal lock mass correction using a background ion (*m*/*z* 445.12003) was applied. For data acquisition and processing of the raw data Xcalibur 4.0 (Thermo Scientific) and Tune version 2.9 were employed. Peptides were separated on the same LC gradient as for the DDA library creation. MS acquisition was performed with the same source settings as for DDA and the following method changes for the data acquisition. Full scan MS spectra with mass range 350-1650 *m*/*z* were acquired in profile mode in the Orbitrap with resolution of 120000. The default charge state was set to 3+. The filling time was set at maximum of 60ms with limitation of 3×10⁶ ions. DIA scans were acquired with 22 (human samples) or 30 (mouse samples) mass window segments of differing widths across the MS1 mass range. HCD fragmentation (stepped normalized collision energy; 25.5, 27, 30%) was applied and MS/MS spectra were acquired with a resolution of 30000 with a fixed first mass of 200 *m*/*z* after accumulation of 3×10⁶ ions or after filling time of 47ms (whichever occurred first). Data were acquired in profile mode.

### 2 Quantification and Statistical Analysis

### 2.1 Global proteome Analysis

For library creation, the DDA and DIA data were searched independently using Pulsar in Spectronaut Professional+ (version 11.0.15038, Biognosys AG, Schlieren, Switzerland). The data were searched against a species specific (*Mus musculus or Homo sapiens*) Swissprot database. The data were searched with the following modifications: Carbamidomethyl (C) (Fixed) and Oxidation (M)/ Acetyl (Protein N-term) (Variable). A maximum of 2 missed cleavages for trypsin were allowed. The identifications were filtered to satisfy FDR of 1 % on peptide and protein level. For each species analysis, a DpD (DDA plus DIA) library was then created by merging the respective DDA and DIA libraries together in Spectronaut. These libraries contained 47291 (mouse); 45357 precursors, corresponding to 3781 (mouse); 3580 protein groups using Spectronaut protein inference. Relative quantification was performed in Spectronaut for each pairwise comparison using the replicate samples from each condition. The data (candidate table) and data reports (protein quantities) were then exported, and further data analyses and visualization were performed with R- studio (version 0.99.902) (http://www.R-project.org/.) using in-house pipelines and scripts.

### 2.2 RNA-Sequencing Analysis

Cells were harvested and resuspended in TRIzol^{™}, and RNA was extracted using the TRIzol-Chloroform method (Heidel *et al.,* 2013). RNA-seq libraries were generated using SMART-Seq v4 Ultra Low Input RNA Kit (Takara Bio USA Inc., Mountain View, CA, USA) followed by Nextera XT (Illumina, San Diego, CA, USA).

For analysis, 50 bp single-end reads (Illumina) were aligned to reference genome mm10 and gtf.-transcriptome annotation release M7 (GENCODE) using STAR two-pass mode (Dobin et al., 2013). GENCODE M7 gtf.-file was used for quantification, performed by STAR, followed by normalization, transformation and differential gene expression analysis in DEseq2 (Love et al., 2014). GSEA was carried out on a public server "Gene Pattern" of Broad Institute (Reich et al., 2006). For analysis of the genetic CRISPRCas9 screen, next generation sequencing was performed on an Illumina NextSeq platform (75bp, single reads) aiming for a minimum of 30Mio reads per sample.

Alignment and statistical analysis of the data was conducted using the MAGeCK and MAGeCK-Flute algorithms as described before (Wang et al., 2019). Specifically, MAGeCK was used to align guide sequences from FASTQ files based on the guide-matrix. Subsequently, the MAGeCK-MLE algorithm was used to statistically compare dropout and enrichment of guides between day 0 and day 12 separately for treated (PR-957) versus untreated (DMSO) conditions. Finally, the FluteMLE algorithm was utilized to identify functional genetic vulnerabilities upon PR-957 treatment based on the MAGeCK-MLE output.

### 2.3 Statistics and analysis of gene expression data

Statistical analyses were performed using Student's t test (normal distribution) or Mann-Whitney U test (when normal distribution was not given). p less than 0.05 was considered statistically significant. mRNA expression data of the catalytic (immuno-) proteasome subunits in different genetic AML subtypes was downloaded from BloodSpot data base (http://servers. binf.ku.dk/bloodspot/) and protein expression data from depmap.org.

**Table 1**

| Key resource table | | |
|---|---|---|
| REAGENT | COMPANY / SOURCE | IDENTIFIER / SEQUENCE |
| Antibodies | | |
| Mouse monoclonal anti-Cas9 | Cell Signaling | Cat# 14697 |
| Mouse monoclonal anti-GAPDH | Meridian Life Sciences | Cat# H86045P |
| Mouse monoclonal anti-HA-Tag | Invitrogen | Cat# 26183 |
| Mouse monoclonal anti-PSMB5 | Santa Cruz Biotechnology | Cat# sc393931 |
| Rabbit monoclonal anti-beta-actin | Cell Signaling | Cat# CS8457 |
| Rabbit monoclonal anti-BASP1 | Invitrogen | Cat# 703692 |
| Rabbit monoclonal anti-c-Myc | Abcam | Cat# ab32072 |
| Rabbit monoclonal anti-FLT3 | Cell Signaling | Cat# 3462 |
| Rabbit monoclonal anti-MEF2C | Cell Signaling | Cat# 5030S |
| Rabbit monoclonal anti-PBX3 | Abcam | Cat# ab109173 |
| Rabbit polyclonal anti-PSMB8/LMP7 | Abcam | Cat# ab3329 |
| Rabbit monoclonal anti-PSMB9/LMP2 | Abcam | Cat# ab242061 |
| Rabbit monoclonal anti-PSMB10/LMP10 | Abcam | Cat# ab183506 |
| HRP-linked anti-mouse IgG | Cell Signaling | Cat# 7076S |
| HRP-linked anti-rabbit IgG | Cell Signaling | Cat# 70743 |
| Biotin anti-mouse Ly-6G (clone RB6-8C5) | BioLegend | Cat# 108403 |
| Biotin anti-mouse/human CD45R (clone RA3-6B2) | BioLegend | Cat# 103203 |
| Biotin anti-mouse Ter119 (clone TER119) | BioLegend | Cat# 116203 |

| REAGENT | COMPANY / SOURCE | IDENTIFIER / SEQUENCE |
|---|---|---|
| Biotin anti-mouse CD3 (clone SK7) | BioLegend | Cat# 344820 |
| Biotin anti-mouse CD4 (clone GK1.5) | BioLegend | Cat# 100403 |
| Biotin anti-mouse CD8 (clone 53-6.7 | BioLegend | Cat# 100703 |
| Biotin anti-mouse CD19 (clone HIB 19) | BioLegend | Cat# 302203 |
| Biotin anti-mouse IL7Ralpha (clone SB/199) | BioLegend | Cat# 135005 |
| Alexa Fluor^{™} 647 Annexin V | BioLegend | Cat# 640912 |
| APC anti-human CD45 | BioLegend | Cat# 304012 |
| APC anti-mouse CD117 (clone 2B8) | BioLegend | Cat# 105812 |
| BV421-Streptavidine (clone H130) | BioLegend | Cat# 405225 |
| FITC anti-mouse CD45.1 (clone A20) | BioLegend | Cat# 110706 |
| PB anti-mouse/human CD11b (clone M1/70) | BioLegend | Cat# 101224 |
| PB SYTOX^{®}Blue Dead Cell Stain | Invitrogen | Cat# S34857 |
| PE anti-mouse Ly-6A/E (clone D7) | BioLegend | Cat# 108108 |
| PE anti-mouse Ly-6G (clone RB6-8C5) | BioLegend | Cat# 108407 |
| PE/Cy7 anti-human CD 13 (clone L138) | BD Biosciences | Cat# 338425 |
| PE/Cy7 anti-mouse CD16/32 (clone 93) | BioLegend | Cat# 101318 |

| Oligonucleotides | | |
|---|---|---|
| shNT | Sigma Aldrich | MISSION^{®} shRNA SHC016 |
| PSMB8 shRNA2 | Sigma Aldrich | MISSION^{®} shRNA TRCN0000007271 |

| REAGENT | COMPANY / SOURCE | IDENTIFIER / SEQUENCE |
|---|---|---|
| PSMB8 shRNA3 | Sigma Aldrich | MISSION^{®} shRNA TRCN0000007269 |
| Luciferase sgRNA | Designed using CRISPick (Broad Insitute) | GATTCTAAAACGGATTACCA (SEQ ID No. 1) |
| RPA3 sgRNA1 | Designed using CRISPick (Broad Insitute) | GGTTGGAAGAGTAACCGCCA (SEQ ID No. 2) |
| RPA3 sgRNA2 | Designed using CRISPick (Broad Insitute) | GATGAATTGAGCTAGCATGC (SEQ ID No. 3) |
| PSMB5 sgRNA1 | Designed using CRISPick (Broad Insitute) | TTTGTACTGATACACCATGT (SEQ ID No. 4) |
| PSMB5 sgRNA2 | Designed using CRISPick (Broad Insitute) | GCTTCATGGAACAACCACCC (SEQ ID No. 5) |
| PSMB5 sgRNA3 | Designed using CRISPick (Broad Insitute) | CCGCTACCGGTGAACCAGCG (SEQ ID No. 6) |
| PSMB5 sgRNA4 | Designed using CRISPick (Broad Insitute) | TGCTTACATTGCCTCCCAGA (SEQ ID No. 7) |
| PSMB8 sgRNA1 | Designed using CRISPick (Broad Insitute) | CCAGAGCTCGCTTTACCCCG (SEQ ID No. 8) |
| PSMB8 sgRNA2 | Designed using CRISPick (Broad Insitute) | AAGGAACGTTCAGATTGAGA (SEQ ID No. 9) |
| PSMB8 sgRNA3 | Designed using CRISPick (Broad Insitute) | CCGGTACTGGCACATCATGT (SEQ ID No. 10) |
| PSMB8 sgRNA4 | Designed using CRISPick (Broad Insitute) | AGGTGCCTTACGGGTGAACA (SEQ ID No. 11) |
| PSMB8 sgRNA5 | Designed using CRISPick (Broad Insitute) | TGTGGCTGGGATAAGAAGGT (SEQ ID No. 12) |
| PSMB9 sgRNA1 | Designed using CRISPick (Broad Insitute) | GTAGATGCGCTCGTGCAGCG (SEQ ID No. 13) |
| PSMB9 sgRNA3 | Designed using CRISPick (Broad Insitute) | TCCCAGGGTTCCATATACCT (SEQ ID No. 14) |
| PSMB9 sgRNA4 | Designed using CRISPick (Broad Insitute) | GCAGCAGCCAAAACAAGTGG (SEQ ID No. 15) |
| PSMB10 sgRNA1 | Designed using CRISPick (Broad Insitute) | CACTAACGATTCGGTCGTGG (SEQ ID No. 16) |
| PSMB10 sgRNA2 | Designed using CRISPick (Broad Insitute) | CGATCAGCGATGCACCCACG (SEQ ID No. 17) |
| PSMB10 sgRNA3 | Designed using CRISPick (Broad Insitute) | TGTGGTCATCTCGGCGTCCG (SEQ ID No. 18) |

| qPCR primers | | |
|---|---|---|
| BASP1_Fw | PrimerBank | aggggaacccaaaaagactga (SEQ ID No. 19) |
| BASP1_Rv | PrimerBank | ggtgtggaactaggcgcttc (SEQ ID No. 20) |
| PSMB5_Fw | Walerych et al., 2016 | GCTACCGGTGAACCAGCG (SEQ ID No. 21) |
| PSMB5_Rv | Walerych et al., 2016 | CAACTATGACTCCAT-GGCGGA (SEQ ID No. 22) |
| PSMB6_Fw | Walerych et al., 2016 | CACTCCAGACTGGGAAAGCC (SEQ ID No. 23) |
| PSMB6 Rv | Walerych et al., 2016 | GACAGCAGAAAATGCGGTCG (SEQ ID No. 24) |
| PSMB7_Fw | Walerych et al., 2016 | CACAGACATGACAACCCAGC (SEQ ID No. 25) |
| PSMB7_Rv | Walerych et al., 2016 | AGGGCTGCACCAATGTAACC (SEQ ID No. 26) |
| PSMB8 _Fw | Origene | CCTTACCTGCTTGGCACCATG (SEQ ID No. 27) |
| PSMB8_Rv | Walerych et al., 2016 | TTGGAGGCTGCCGACACT-GAA (SEQ ID No. 28) |
| PSMB9_Fw | Walerych et al., 2016 | GGCGTTGTGATGGGTTCTGA (SEQ ID No. 29) |
| PSMB9_Rv | Walerych et al., 2016 | AGAGAGTGCACAGTAGAT-GCG (SEQ ID No. 30) |
| PSMB10_Fw | Walerych et al., 2016 | AGCTACACGCGTTATCTACGG (SEQ ID No. 31) |
| PSMB10_Rv | Walerych et al., 2016 | CTGCGGTCCAGTCAGGTCTA (SEQ ID No. 32) |
| Beta-2-microgobulin_Fw | In stock | TGTGTCTGGGTTTCA-TCCATCCGA (SEQ ID No. 33) |

| REAGENT | COMPANY / SOURCE | IDENTIFIER / SEQUENCE |
|---|---|---|
| Beta-2-microgobulin_Rv | In stock | CACACGGCAGGCATACTCA-TCTTT (SEQ ID No. 34) |

### 3 Experimental data and results

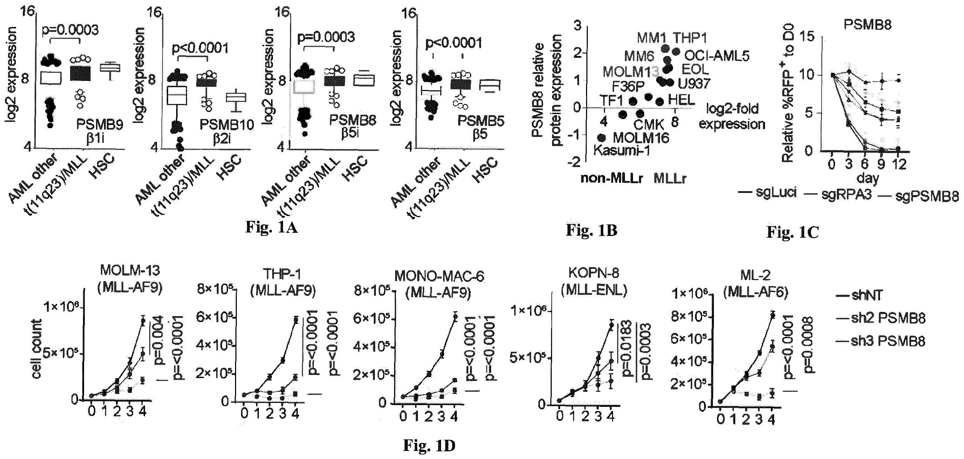

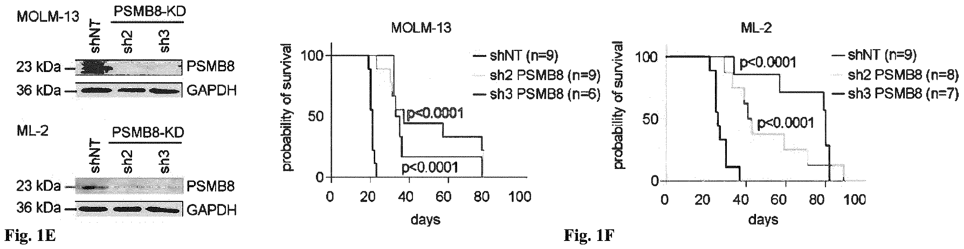

To investigate the expression of relevant catalytic proteasome subunits, transcriptional regulation was analysed in published datasets (Wouters et al., 2009) as well as the publicly available database bloodspot (www.bloodspot.eu) (Bagger et al., 2018). Here, catalytic standard proteasome subunit PSMB5 showed higher expression in MLLr leukemias compared to all other genetic subgroups (Fig. 1A). Even more pronounced, catalytic immunoproteasome (IP) subunits PSMB8, PSMB9 and PSMB10 (corresponding to LMP7, LMP2 and LMP10/MECL1 in the murine system) showed significantly higher expression levels in MLLr AML (Fig. 1A) compared to non-MLL-AMLs. This could be confirmed in proteome data from primary human AML cell lines, specifically for PSMB8 (Fig. 1B). In order to assess the functional importance of catalytic immunoproteasome subunit PSMB8 in MLL-rearranged leukemia, we used a CRISPR-Cas9 mediated genome editing and negative selection strategy in MLLr MOLM13 cells (Fig. 1C). When using a stable and blasticidin selected MOLM13-Cas9 cell line, consistent outcompetition of PSMB8 (3/5 sgRNAs) depleted cells was observed against non-infected competitors.

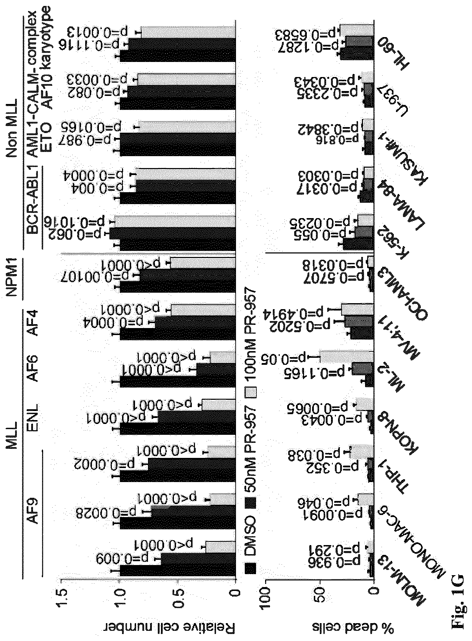

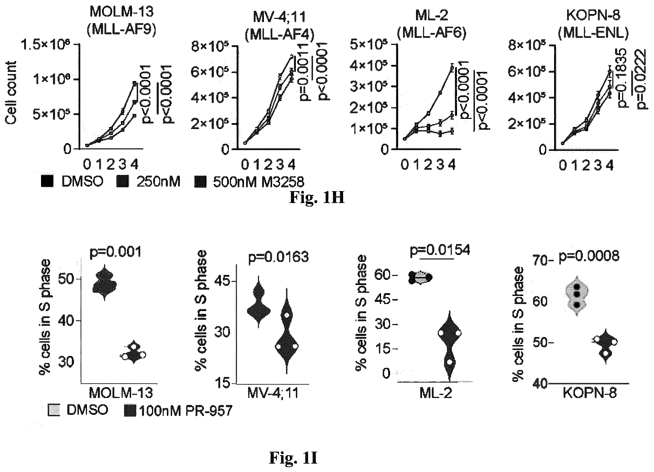

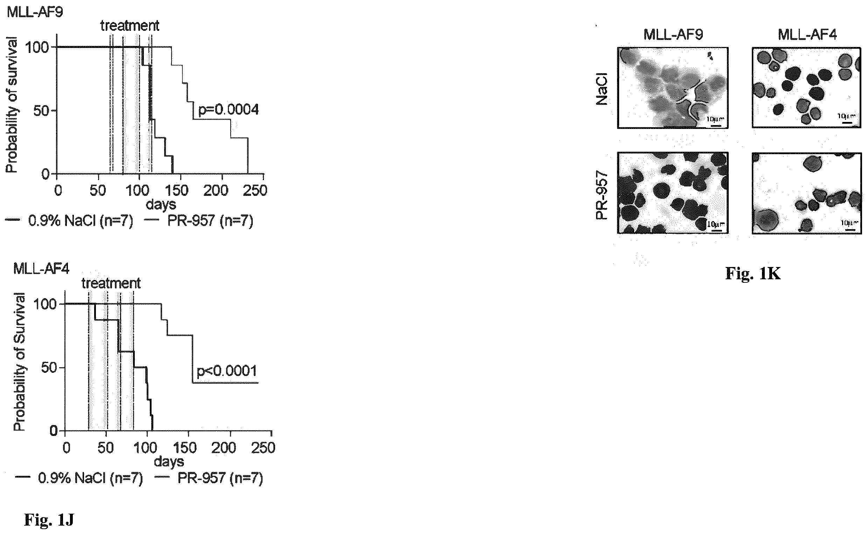

In order to assess for the functional relevance of immunoproteasome component LMP7/PSMB8 in human AML, genetic inactivation of LMP7/PSMB8 by RNAi was performed with two independent shRNAs against LMP7/PSMB8 (shPSMB8-3 and shPSMB8-2) compared to non-targeting control (shNT) in MLLr cell lines. Cell counts assessed on days 1-4 revealed attenuated cell growth in LMP7/PSMB8 depleted cells (Fig. 1D). However, induction of apoptosis could only be detected in less than 40% of cells with no significant variability between MLLr cell lines and the individual shRNAs. Ewfficient LMP7/PSMB8 depletion was confirmed by Western blotting (Fig. IE). To corroborate the findings and to confirm the functional relevance of LMP7/PSMB8 *in vivo,* equal numbers of LMP7/PSMB8 depleted and (non-targeting) control (ML-2 or MOLM-13) cells were injected into pre-conditioned immuno-compromised mice. Recipients of LMP7/PSMB8 deficient cells revealed delay in disease development and prolonged survival compared to NT-control (Fig. 1F). Together, these results suggest a critical requirement of LMP7/PSMB8 for proliferation and clonogenicity of human MLLr AML cells. To test whether this specific dependency is attributed to the loss of LMP7/PSMB8 protein expression or rather its function within the immunoproteasome, again the LMP7/PSMB8 specific inhibitor PR-957 (Muchamuel et al., 2009) was used to inactivate its function in an extended panel of MLLr and non-MLLr cell lines. Comparable to the effects observed after genetic inactivation of LMP7/PSMB8, pharmacologic inhibition resulted in dose-dependent and specific reduction of cell growth in MLLr cell lines (Fig. 1G, left), while leaving non-MLLr cell lines largely unaffected (Fig. 1G, right). These effects could be recapitulated with a second and novel immunoproteasome inhibitor (M3258) at concentrations of 250-500nM in MLL-rearranged human AML cell lines (Fig. 1H). Also, pharmacologic immunoproteasome inhibition resulted in reduced cell cycle activity of human MLL-rearranged AML cell lines (Fig. 1I). On the basis of the susceptibility of human MLLr AML cells, the hypothesis that this subtype of AML may be susceptible to immunoproteasome inhibition was tested next. Primary leukemic blasts derived from two different patients harboring MLL-AF9 and MLL-AF4 fusions, respectively, were transplanted into NOD.Cg^{KitW-41J}Prkdc^{scid}Il2rg^{tm1Wjl} (NSGW41) animals. Following xenotransplantation, the relative abundance of human AML cells in the murine peripheral blood was measured over time by flow cytometry, and PR-957 treatment was initiated once hCD45+ AML cells constituted 0.1%-0.5% of PB cells. PR-957 treatment was initiated at 3mg/KG i.v. for 5 consecutive days and continued every 2 weeks with dose escalations of 6mg/KG and finally 10mg/KG. In this PDX models that provides a higher competitive pressure against the expansion of leukemic cells due to the lack of pre-conditioning, PR-957 exhibited potent anti-leukemic activity and resulted in loss of competitive advantage. While MLLr leukemic cells engrafted at equal numbers, they failed to expand and outcompete the murine hematopoiesis and niche cells, when treated with PR-957. This loss of competitive advantage in a minimal residual disease situation resulted in improved survival and reduced disease penetrance over a period of 100 days (Fig. 1J). AML cells exposed to PR-957 treatment showed signs of cellular differentiation in cytospin analysis (Fig. 1K).

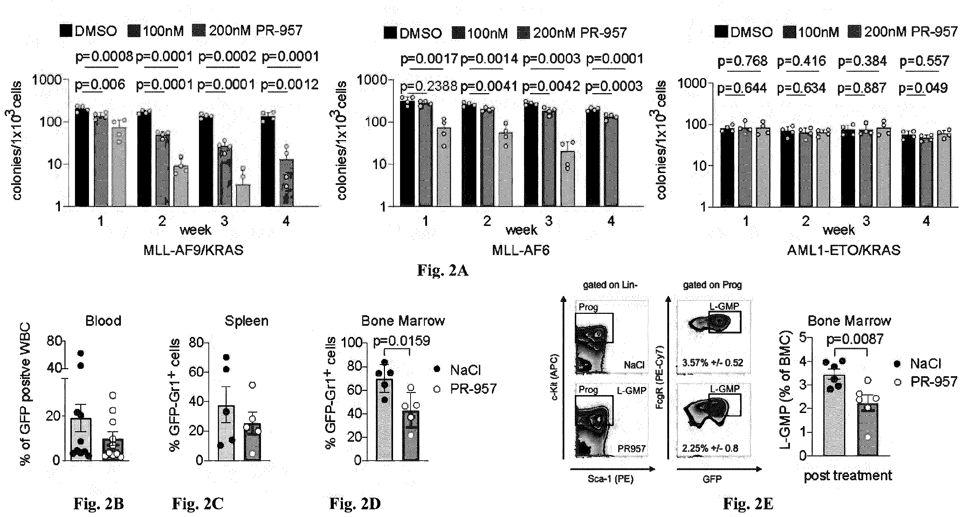

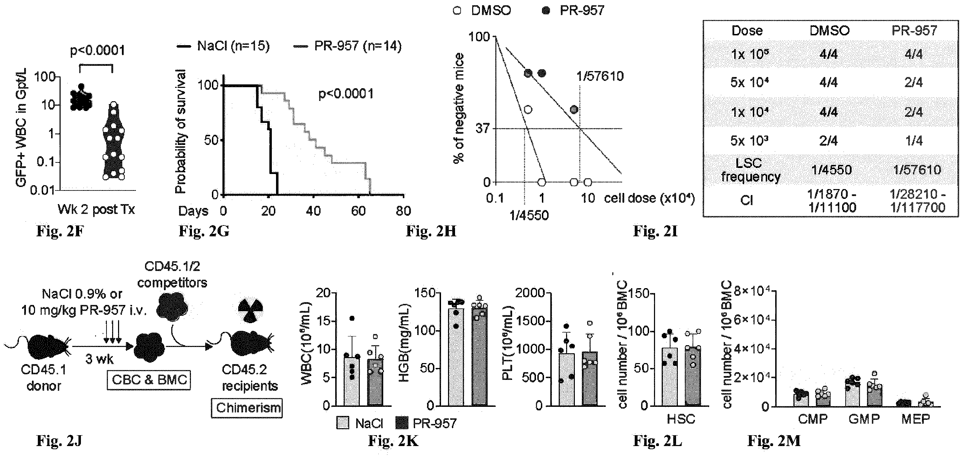

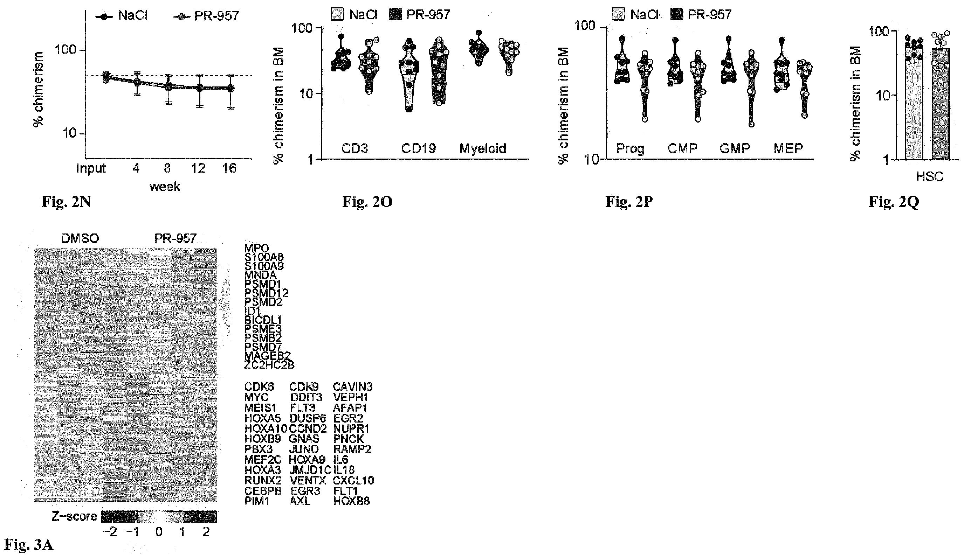

Exposure of MLL-rearranged murine leukemic cells to PR-957 at nanomolar concentrations resulted in profound dose dependent reduction of clonogenic potential (Fig. 2A) which was not detectable when treating non-MLL fusion oncogenes such as AML1-ETO (Fig. 2A, right panel). Moreover, treatment with PR-957 resulted in reduction of colony size and changes in colony shape, specifically in MLL-AF9 transformed leukemia. To characterize the effects of LMP7/PSMB8 inhibition in more detail, differentiation following PR-957 treatment was assessed. As leukemic cells are highly heterogeneous, it was also aimed to assess for the leukemic stem cell (LSC) pool, that is inherently resistant to chemotherapy, frequently persists after treatment and is the source of clinical relapse. Murine HSPCs were transduced with MLL-AF9 to induce leukemia in sublethally irradiated primary recipient mice to maintain competitive pressure by an incompletely cleared niche. Primary recipients were treated with 6mg/kg PR-957 i.v. QD for 3 weeks (5 days per week) and PB and BM were investigated 3 days after treatment discontinuation. Leukemic cells in the peripheral blood and spleen size of PR-957 treated animals were reduced compared to diluent treated controls (Fig. 2B,C). Consistently, significant decrease was found in the abundance of leukemic cells in the bone marrow of PR-957 treated mice (42.9% versus 69.8%; p=0.0159^{∗}; Fig. 2D). Next, the frequency of L-GMP (Lin- c-kit+ Sca1- CD34+ FcgR+) was assessed by flow cytometry, which have been described as the functionally relevant LSC population in MLL-AF9 driven leukemia. Of note, PR-957 treated animals showed significant reduction of L-GMP populations compared to diluent treated controls (2.25+/-0.8% and 3.57+/-0.52%, respectively, p=0.0087^{∗∗}; Fig. 2E). Injection of 1×10⁶ total bone marrow cells into sublethally irradiated secondary recipient mice resulted in decreased numbers of leukemic cells in the peripheral blood two weeks after transplantation (17.2 Gpt/L versus 1.6Gpt/L GFP+ WBC; p<0.0001^{∗∗∗∗}; Fig. 2F) and significant delay in disease development for recipients of PR-957 treated cells (median survival 39.5 days versus 21 days; p<0.0001^{∗∗∗∗}; Fig. 2G). To determine the functional abundance of LSCs and the leukemia initiating potential, limiting dilution assays were performed by injecting sublethally irradiated recipient mice (7Gy) with limiting numbers (1×10⁵, 5×10⁴, 1×10⁴, 5×10³) of GFP+ leukemic bone marrow cells (Fig. 2H,I). PR-957 treated cells showed profound, more than log-fold reduction in LSC frequency compared to diluent treated controls (1/57610, CI 1/1870-1/11100 versus 1/4550; CI 1/28210-1/117700). Taken together, pharmacologic immunoproteasome inhibition using the specific LMP7/PSMB8 inhibitor PR-957 attenuated the leukemia initiating potential of MLL-rearranged AML cells *in vivo* and this effect was even more pronounced in the leukemia stem cell (L-GMP) population. Improved survival could therefore be explained by both, reduced fitness and decreased LSC numbers. To be considered a valid therapeutic option, immunoproteasome inhibition must have a beneficial therapeutic index between hematopoietic stem- and progenitor cells (HSPCs) and the respective leukemic counterpart. This is particularly important in considering that immunoproteasome subunits such as LMP7 are highly expressed in normal HSPCs compared to mature cell compartments. Treatment of normal hematopoietic cells following competitive transplantation against CD45.1/2 competitors (Fig. 2J) with PR-957 for 3 weeks did not show any changes in hematopoietic stem- and progenitor cell abundance (Fig.s 2K,L,M). When transplanted into secondary recipient mice to test for HSC function, no changes in peripheral blood chimerism could be detected until week 16 (Fig. 2N). Likewise, stem- and progenitor cell chimerism in secondary recipients was not affected by previous PR-957 treatment (Fig.s 2O,P,Q).

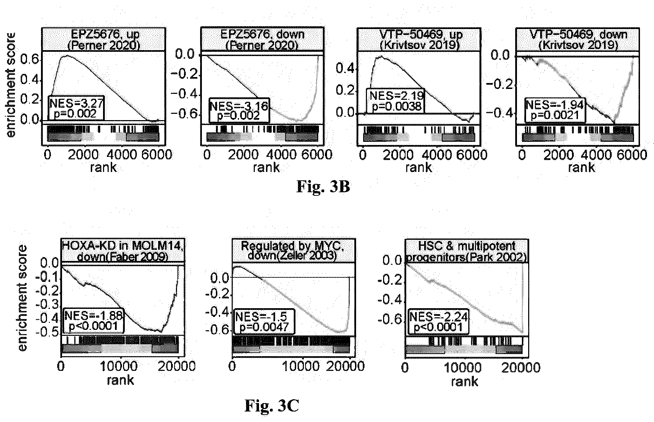

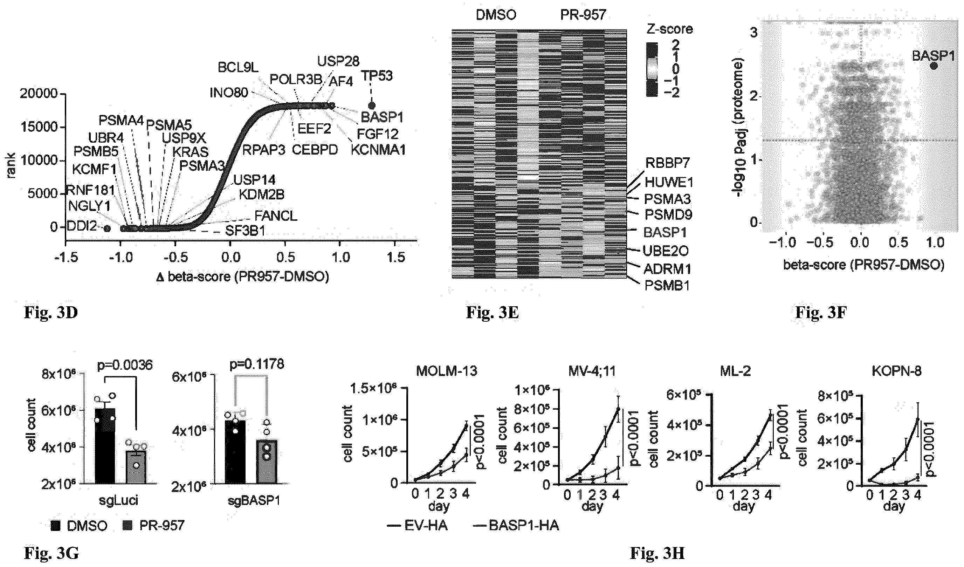

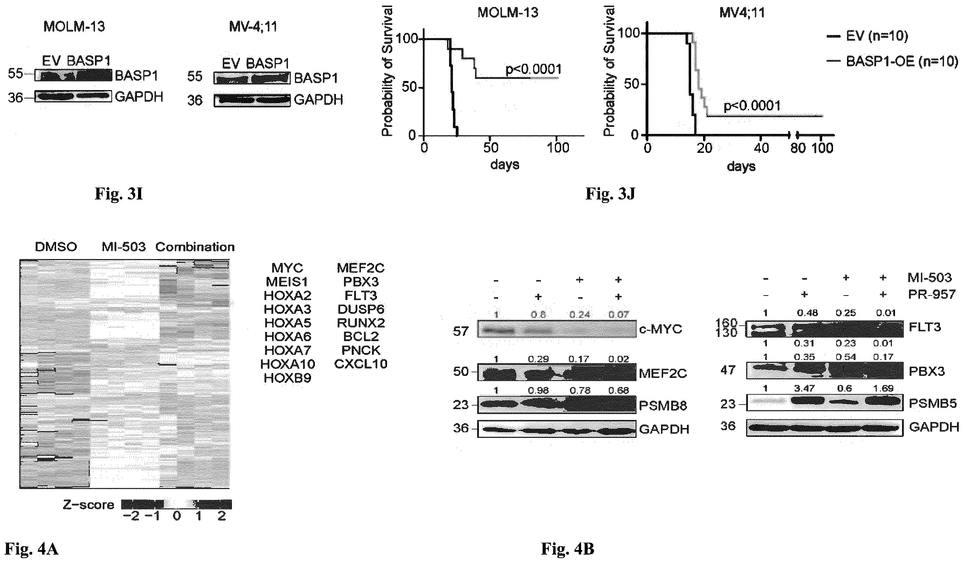

According to the functional data it was found that PSMB8/LMP7-dependent immunoproteasome function is required for the development and maintenance of human MLLr leukemia. Therefore, it was sought to determine to which extent the inhibition of PSMB8/LMP7 also contributes to the transcriptional regulation of human MLL-target genes. Global transcriptome analysis by RNA-sequencing performed in MOLM-13 and ML-2 cells revealed overlap of 457 genes regulated in both cell lines upon PR-957 treatment (Fig. 3A). Gene Set Enrichment Analysis (GSEA) showed that Hox-gene, Myc- and sternness-related gene-sets were enriched within the PSMB8/LMP7-signature (Fig. 3C). Intriguingly, downstream effector genes of MLL-fusions and those deregulated by epigenetic inhibitors of the MLL-complex (e.g. Dot1L- or Menin-inhibitors) were enriched in the gene sets regulated by pharmacologic PSMB8/LMP7 inhibition (Fig. 3B). These findings indicated a partial overlap of MLL-fusion target gene regulation between MLL-complex inhibitors (Menin, Dot1L) and immunoproteasome (PSMB8/LMP7) inhibition (Fig. 3A). In order to identify functional dependencies on the immunoproteasome subunit LMP7/PSMB8, a genome-wide CRISPR-Cas9 screen was applied in the human MLLr cell line MOLM13. This cell line was selected for its sensitivity to PR-957 (Fig.3D) and ability to be transduced among the MLLr cell lines evaluated above. For the screen, MOLM-13 cells were infected with the human Liu lentiviral library, containing 92817 single guide RNAs targeting against 18436 genes and treated with PR-957 (or DMSO, as control) for 12 days. Alignment and statistical analysis of the data was performed using the MAGeCK and MAGeCK-Flute algorithms as described before (Wang et al., 2019). Specifically, MAGeCK was used to align guide sequences from FASTQ files based on the guide-matrix. Subsequently, the MAGeCK-MLE algorithm was used to statistically compare dropout and enrichment of guides between day 0 and day 12 separately for treated (PR-957) versus untreated (DMSO) conditions. Finally, the FluteMLE algorithm was utilized to identify functional genetic vulnerabilities upon PR-957 treatment based on the MAGeCK-MLE output. This pipeline offers the opportunity to specifically identify synthetic lethal hits and resistance mediators, as well as genes that are potential downstream effectors of the drug target. This functional screen identified resistance mediators to PR-957 treatment, including TP53, BASP1 and AF4 (Fig. 3D). Out of the resistance mediators only BASP1 was among the proteins that showed increased abundance upon PR-957 inhibition in global proteome analysis (Fig.s 3E,F). Deletion of BASP1 rescued the PR-951induced phenotype (Fig. 3G). Conversely, overexpression of BASP1 (Fig. 31) resulted in reduced proliferative capacity (Fig. 3H) *in vitro* and delayed disease development of human MLLr-AML cells *in vivo* (Fig. 3J), recapitulating the PR-957 induced phenotype.

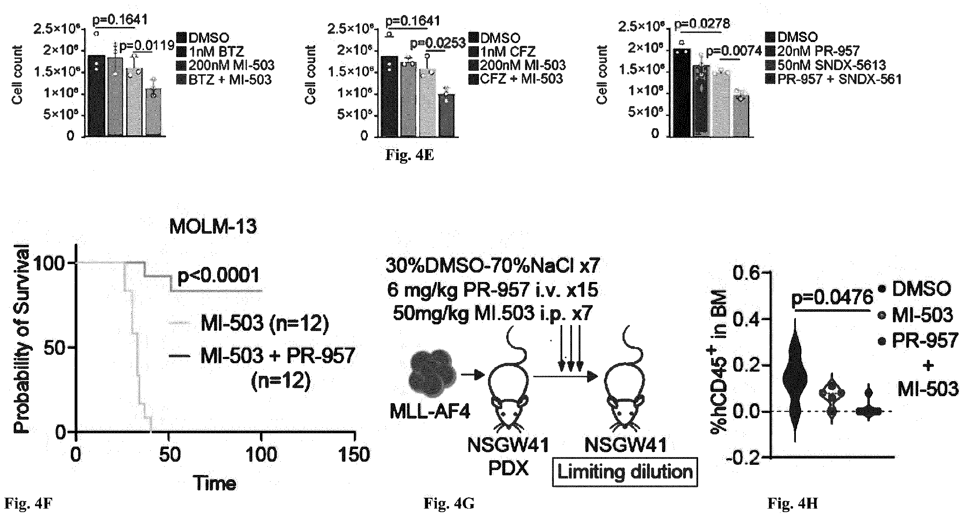

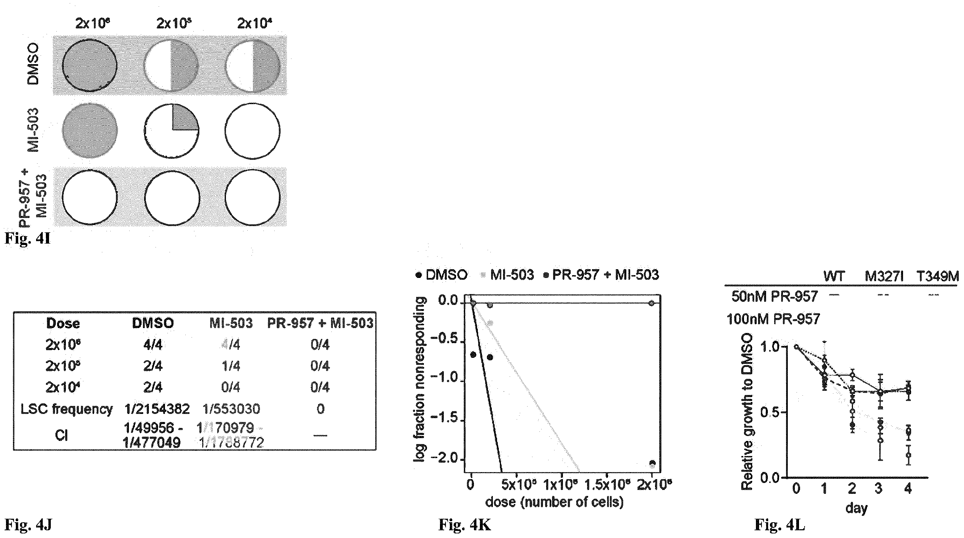

As pharmacologic inhibition of PSMB8/LMP7 has shown efficacy in reducing oncogene induced transcriptional targets, it was aimed to assess for **additive/synergistic effects of combinatorial treatments** with inhibitors of the MLL-complex (e.g. Menin-inhibitors). Combination of Menin-inhibitor MI-503 with immunoproteasome inhibitor PR-957 resulted in more pronounced reduction of MLL-dependent target gene expression (Fig. 4A). Combinatorial effects could be confirmed for MLL-targets c-MYC, MEF2C, FLT3 and PBX3 also on the protein level (Fig. 4B). Combination of Menin-inhibitor MI-503 with either immunoproteasome inhibitor (PR-957 or M3258) resulted in decreased proliferative capacity of MLLr- or NPM1-mutated human AML cell lines (as compared to single agent therapy) (Fig.s 4C-E). Consistently, combination of PR-957 with MI-503 reduced disease penetrance by >80% in a MOLM-13 driven human xenograft model of AML (p<0.0001, Fig. 4F).

In a limiting dilution assay primary human leukemia cells harboring an MLL-AF4 fusion were used for a patient-derived xenograft model (Fig.s 4G-K). Treatment with a combination of PR-957 with MI-503 *in vivo* resulted in significant reduction of leukemic cells (Fig. 4G) and reduction of leukemia initiating cells by factor 25-30 (Fig.s 41, J, K). These findings highlighted the potency of the combination to eradicate leukemia stem cells that are the relevant population responsible for clinical relapse. Most recently, Menin-inhibitor treatment has been investigated in early clinical trials for MLLr- and NPM-mutated AML (Stein et al. ASH 2021). While promising responses could be documented, they were rather short-lived and resistance developed rapidly. Most recently, mutations in Menin could be identified *in vitro* and *in vivo* as the main reason for Menin-inhibitor resistance (Perner et al. 2022, unpublished). Of note, addition of the immunoproteasome inhibitor PR-957 to Menin-inhibitor resistant cell lines (harboring resistance mutations M327I and T349M) showed preserved sensitivity of these cells to immunoproteasome inhibition (Fig. 4L). This finding indicated a potent combination therapy that prevents development of resistance and improves and already compelling targeted therapy (Menin-inhibitor therapy).

### Description of Figures

**Fig. 1A** shows a boxplot depicting the mRNA expression levels of the (immuno-) proteasome subunits PSMB10, PSMB9, PSMB8 and PSMB5 in patients bearing a t(11q23)/MLL leukemia (n=43) compared to other AML subtypes (n=209) and normal hematopoietic cells (HSC) (n=6). Statistical analysis was performed using the Mann-Whitney U test.
**Fig. 1B** shows the PSMB8 relative protein expression among MLLr and non-MLLr cell lines from the cancer cell line encyclopedia (Mass-spec proteome analysis, data derived from depmap.org).
**Fig. 1C** shows a line graph showing the % of RFP+ cells from a CRISPR-Cas9 cell competition assay. PSMB8 was knocked out by single guide RNAs in MOLM13- Cas9 expressing cells and the chimerism of knockout and wildtype cells at day 0, 3, 6, 9 and 12 was visualized in the graphs. n=6 independent experiments.
**Fig. ID** shows growth curves depicting cell counting after trypan blue exclusion of MOLM-13, THP-1, MONO-MAC-6, KOPN-8 and ML-2 cells transduced with shRNAs targeting PSMB8 or a non-targeting control (shNT). n=3 independent experiments, in triplicate; statistical analysis was performed using 2-way ANOVA.
**Fig. 1E** shows a Western blot confirming PSMB8 depletion on the protein level after shRNA knock-down in MOLM-13 and ML- 2 cells. Samples were collected 72 hours after transduction and before transplantation into recipient mice.
**Fig. 1F** shows Kaplan-Meier survival curves of NSG or NSGS recipient mice transplanted with 1×105 MOLM-13 or ML-2 cells, respectively, expressing shRNA2 (n=9 for MOLM-13; n=8 for ML2) and shRNA3 (n=6; n=7) against PSMB8 or shNT (n=9; n=9). Shown are two independent cohorts; statistical analysis was performed using the Mantel-Cox test.
**Fig. 1G** shows the celative cell number and percentage of death cells (measured as SYTOX^{®}Blue+ cells by Fluorescence-Activated Cell Sorting (FACs)) of human leukemic cell lines containing an MLL rearrangement, NPM1 mutated or non- MLL rearranged after treatment for 96 hours with 50nM or 100nM PR-957 or DMSO as a control. n=4 independent experiments, in triplicate; statistical analysis was performed using a paired t test.
**Fig. 1H** shows growth curves depicting cell counts of MOLM-13, MV-4;11, ML-2 and KOPN-8 cells after treatment with 250nM or 500nM M3258 or DMSO as a control. n=4 independent experiments; 2- way ANOVA.
**Fig. 1I** shows violin plots showing the % of cells detected in S phase in MOLM-13, MV-4;11, ML-2 and KOPN-8 cells after treatment with 100nM PR-957 or DMSO for 24 hours. Samples were labeled for FACs based analysis of cell cycle using the Click-iTR EdU assay. n=3 independent experiments; paired t test.
**Fig. 1J** shows Kaplan-Meier survival curves for two patient derived xenografts (PDX) using 1-5x104 cells from patient samples containing an MLL-AF9 or MLL-AF4 translocation that were injected into NSGW41 recipient mice. After engraftment, mice were treated *in vivo* with 3- 6m/kg PR-957 for 5 days/week on 4 alternate weeks (n=7 for both PDX models) or NaCl 0.9% as diluent control (n=7 for both PDX models). Two independent cohorts; Mantel-Cox test.
**Fig. 1K** shows Cytospins (May-Grunwald/Giemsa staining) showing cell morphology of MLL-AF9 and MLL-AF4 PDX after onset of AML in recipient mice treated with PR-957 or diluent control.
**Fig. 2A** shows Colony counts over 4 weeks after serial replating in methylcellulose of MLLAF9/ KRAS, MLL-AF6 and AML1-ETO/KRAS transduced primary murine cells treated with 100nM or 200nM PR-957 or DMSO as a control. n=3-4 independent experiments; multiple paired t tests.
**Fig.s 2B-2E** show 3x105 MLL-AF9 murine whole bone marrow cells were transplanted into recipient mice that were treated for 5 days/week per 3 weeks with 10 mg/kg PR-957 (n=10) or NaCl 0.9% as diluent control (n=10). Immunophenotyping of GFP+ white blood cells (WBC) in blood (B), GFP+-Gr1+ cells in spleen (C) and bone marrow (D) and leukemic granulocyte-macrophage-progenitors (LGMPs) defined as Lin-cKit+Sca-1-GFP+ cells (E) (left panel: representative FACs plot with the gating strategy; right panel: bar graph showing the % of L-GMPs in the whole bone marrow). Two independent cohorts; Mann-Whitney U test.
**Fig.s 2F-2G** show 2×10⁶ whole bone marrow cells from the PR-957 or NaCl *in vivo* treated MLL-AF9 mice were transplanted into secondary recipients (n=24 recipients of PR957 treated mice; n=25 recipients of NaCl treated mice). Immunophenotyping of GFP+ WBC two weeks after transplantation (F) and survival curves (G). Two independent cohorts; Mann- Whitney U test and Mantel-Cox test respectively.
**Fig.s 2H-2I** shows a limiting dilution assay that was performed by injecting MLL-AF9 leukemic whole bone marrow cells treated *ex vivo* with 200nM PR-957 or DMSO as a control for 48 hours into recipients hosts in limiting numbers (1×105, 5x104, 1x104, 5x103; n=4 per dilution and treatment). Number of engrafted mice per dilution is shown and leukemic stem cell (LSC) frequency calculated using the ELDA (Extreme Limiting Dilution Assay) software (reference; Hu et al., 2009). LSC frequency was 1/4550 for DMSO treated recipients (95% confidence interval, 1/1870-1/11100) and 1/57610 for PR-957 treated recipients (95% confidence interval, 1/28210-1/117700).
**Fig. 2J** shows a schematic representation of the *in vivo* PR-957 (10mg/kg) or NaCl 0.9% treatment for 5 days/week per 3 weeks in CD45.1 mice and subsequent transplantation of 2x106 cells in a competitive manner with 2×10⁶ CD45.1/2 competitor cells into lethally irradiated CD45.2 recipient mice.
**Fig. 2K** shows levels of white blood counts (WBC), hemoglobin (HGB) and platelets (PLT) in peripheral blood of CD45.1 mice treated with PR-957 (n=6) or NaCl (n=6).
**Fig. 2L** shows the number of hematopoietic stem cells (HSCs) (Lin-cKit+Sca-1+CD34-) per 106 bone marrow cells (BMCs).
**Fig. 2M** shows the number of common myeloid progenitors (CMP) (Lin-cKit+Sca-1-CD34+FcγR-), granulocyte macrophage progenitors (GMP) (Lin-cKit+Sca-1-CD34+FcγR+) and macrophage erythrocyte progenitors (MEP) (Lin-cKit+Sca-1-CD34-FcγR-) per 106 BMCs.
**Fig. 2N** shows peripheral blood chimerism of recipient mice (NaCl, n=10; PR-957, n=10) over 16 weeks; two independent cohorts.
**Fig. 2O** shows immunophenotypic quantification of T-lymphoid (CD3), B-lymphoid (CD19) and mature myeloid bone marrow cells (NaCl, n=10; PR-957, n=10).
**Fig. 2P** shows immunophenotypic quantification of progenitor cell abundance: progenitors (prog; LK (Lin-cKit+), CMP, GMP and MEP (NaCl, n=10; PR-957, n=10).
**Fig. 2Q** shows immunophenotypic quantification of HSCs (NaCl, n=10; PR-957, n=10).
**Fig. 3A** shows a eatmap of differentially expressed genes between MOLM-13 cells treated for 72 hours with 100nM PR-957 and DMSO treated cells. Upregulated genes are depicted in red and downregulated genes in blue.
**Fig. 3B** shows a Gene Set Enrichment Analysis (GSEA) indicating enrichment for upregulated genes after EPZ5676 or VTP-50469 treatment and negative enrichment of downregulated genes after EPZ5676 or VTP-50469 treatment in PR-957 treated cells; NES, normalized enrichment score.
**Fig. 3C** shows a Gene Set Enrichment Analysis (GSEA) indicating loss of HOXA9 and MYC target genes and loss of a signature of genes commonly expressed in HSCs and progenitor cells compared to mature lineage cells; NES, normalized enrichment score.
**Fig. 3D** shows a dot-plot of ranked genes found to be differentially enriched or depleted after knock-out between MOLM-13 cells treated for 12 days with increasing concentrations (50 to 200nM) of PR-957 and DMSO treated cells in the CRISPR-Cas9 screening. The X axis shows the differential Δbeta-scores and the Y axis the gene-rank.
**Fig. 3E** shows a heatmap derived from unsupervised hierarchical clustering of differentially abundant proteins detected by mass-spectrometry between MOLM-13 cells treated for 72 hours with 100nM PR-957 and DMSO treated cells.
**Fig. 3F** shows the correlation between the magnitude of differential protein abundance after PR-957 treatment and the dependency of the respective genes (Δbeta-scores between PR-957 and DMSO treated cells) in the CRIPSR-Cas9 screening.
**Fig. 3G** shows bar plots depicting proliferation in MOLM-13-Cas9 cells treated for 4 days with 100nM PR-957 or DMSO. The treatment was started 6 days after transduction with an sgRNA against BASP1 or sgLuciferase as a non-targeting control. n=4 independent experiments, in triplicate; paired t test.
**Fig. 3H** shows growth curves of MOLM-13, MV-4;11, ML-2 and KOPN-8 transduced with a pLEX vector containing the sequence of BASP1 and an HA tag (BASP1-HA) or an empty vector with the HA tag (EV-HA) as a control. n=3 independent experiments, in triplicate; 2-way ANOVA.
**Fig. 3I** shows western blot confirming BASP1 overexpression on the protein level after transduction with pLEX-BASP1 or pLEX-EV in MOLM- 13 and MV-4;11 cells. Samples were collected 96 hours after transduction and before transplantation into recipient mice.
**Fig. 3F** shows Kaplan-Meier survival curves of NXG recipient mice transplanted with 1×105 MOLM-13 or MV-4;11 cells expressing pLEX-BASP1 or pLEX-EV (n=10 per construct and cell line). Two independent cohorts; Mantel-Cox test.
**Fig. 4A** shows a heatmap of differentially expressed genes between MOLM-13 cells treated with 1µM MI-503 ("MI-503"), with a combination of 1µM MI-503 and 100nM PR-957 ("Combination") or with DMSO ("DMSO") for 72 hours. Upregulated genes are depicted in red and downregulated genes in blue.
**Fig. 4B** shows representative Western blot plots showing expression of c-Myc, MEF2C, FLT3, PBX3, PSMB5 and PSMB8 on the protein level in MOLM-13 cells treated with 1µM MI-503, with a combination of 1µM MI-503 and 100nM PR-957 or with DMSO for 72 hours.

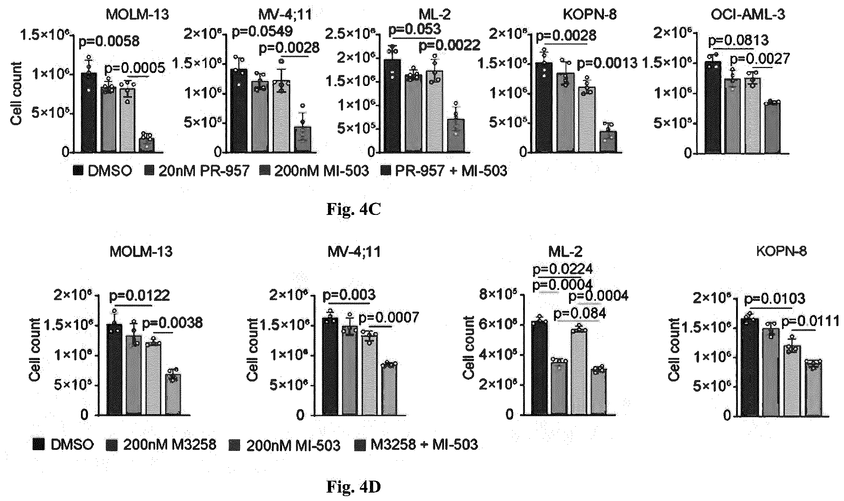

**Fig. 4C** shows bar plots depicting cell counts in MOLM-13, MV-4;11, ML-2, KOPN-8 and OCI-AML3 cells after treatment with 20nM PR-957, 200nM MI-503, a combination of both or DMSO as a control for 6 days. n=5 independent experiments; paired t test.
**Fig. 4D** shows bar plots showing proliferation in MOLM-13, MV-4;11, ML-2 and KOPN-8 after treatment with 200nM M3258, 200nM MI-503, a combination of both or DMSO as a control for 6 days. n=4 independent experiments; paired t test..
**Fig. 4E** Bar plots representing proliferation in MOLM-13 cells after treatment with 2nM bortezomib (BTZ) / 2nM carfilzomib (CFZ) / 20nM PR-957, 200nM MI-503 / 50nM SNDX-5613, different combinations as indicated or DMSO as a control for 6 days. n=3 independent experiments; paired t test.
**Fig. 4F** shows the survival curve of NXG recipient mice transplanted with 1×105 MOLM-13 cells previously treated *ex vivo* with 2,5 µM MI-503 for 96 hours or a combination of 2,5 µM MI-503 for 96 hours and 100nM PR-957 for 48 hours (n=12 per treatment). Three independent cohorts; Mantel-Cox test.
**Fig. 4G** shows a schematic representation of the *in vivo* PR-957 (6mg/kg, 5 days/week per 3 weeks), MI-503 (50mg/kg, x7 times) or 30%DMSO-70%NaCl (x7 times) treatment ofNSGW41 mice (n=5 per treatment) injected with 2×104 whole bone marrow cells from a patient derived xenograft (PDX) of a patient harboring a MLL-AF4 rearrangement. Whole bone marrow cells were subsequently transplanted in limiting numbers (2×106, 2x105, 2x104) into NSGW41 recipient mice (n=4 per dilution and treatment).
**Fig. 4H** shows immunophenotyping of human CD45+ (hCD45+) cells in the bone marrow of NSGW41 mice after *in vivo* treatment with MI-503, the combination of MI-503 and PR-957 or a dilution of DMSO in NaCl. n=5 mice per treatment; Mann- Whitney U test.
**Fig. 4I** shows percentages of mice with >1% of hCD45+ cells in peripheral blood (grey) or <1% (white), three months after transplantation of whole bone marrow cells from the *in vivo* PDX treated mice with DMSO-NaCl, MI-503 or the combination of MI-503 with PR-957 into NSGW41 recipient mice.
Fig.s 4J-4K show number of engrafted mice per dilution (engraftment is considered if the percentage of hCD45+ cells is >1% in peripheral blood three months after transplantation) is shown and leukemic stem cell (LSC) frequency calculated using the ELDA (Extreme Limiting Dilution Assay) software (reference). LSC frequency was 1/247973 for DMSO treated recipients (95% confidence interval, 1/71606-1/858736), 1/363914 for MI-503 treated recipients (95% confidence interval, 1/101632-1/1303062) and 1/8783601 for the recipients treated with the combination of MI-503 and PR-957 (95% confidence interval, 1/1125784-1/68531515).
**Fig. 4L** shows relative growth to DMSO of wild-type MV- 4;11 (MV-4;11 WT) and two MV-4;11 cell lines containing two different mutations in KMT2A (MV-4;11 M327I, contains a change from methionine to isoleucine at position 327; MV-4;11 T349M, contains a change from threonine to methionine at position 349) after treatment with 50nM or 100nM PR-957. n=4 independent experiments; 2-way ANOVA.

### Literatur

- Bernt, K.M., Zhu, N., Sinha, A.U., Vempati, S., Faber, J., Krivtsov, A.V., Feng, Z., Punt, N., Daigle, A., Bullinger, L., et al. (2011). MLL-rearranged leukemia is dependent on aberrant H3K79 methylation by DOT1L. Cancer Cell 20, 66-78. 10.1016/j.ccr.2011.06.010.
- Cosgun, K.N., Rahmig, S., Mende, N., Reinke, S., Hauber, I., Schafer, C., Petzold, A., Weisbach, H., Heidkamp, G., Purbojo, A., et al. (2014). Kit regulates HSC engraftment across the human-mouse species barrier. Cell Stem Cell 15, 227-238. 10.1016/j.stem.2014.06.001.
- Dobin, A., Davis, C.A., Schlesinger, F., Drenkow, J., Zaleski, C., Jha, S., Batut, P., Chaisson, M., and Gingeras, T.R. (2013). STAR: ultrafast universal RNA-seq aligner. Bioinformatics 29, 15-21. 10.1093/bioinformatics/bts635.
- Doench, J.G., Hartenian, E., Graham, D.B., Tothova, Z., Hegde, M., Smith, I., Sullender, M., Ebert, B.L., Xavier, R.J., and Root, D.E. (2014). Rational design of highly active sgRNAs for CRISPR-Cas9- mediated gene inactivation. Nat Biotechnol 32, 1262-1267. 10.1038/nbt.3026.
- Fehling, H.J., Swat, W., Laplace, C., Kuhn, R., Rajewsky, K., Muller, U., and von Boehmer, H. (1994). MHC class I expression in mice lacking the proteasome subunit LMP-7. Science 265, 1234-1237. 10.1126/science.8066463.
- Gautier, L., Cope, L., Bolstad, B.M., and Irizarry, R.A. (2004). affy--analysis of Affy-metrix GeneChip data at the probe level. Bioinformatics 20, 307-315. 10.1093/bioinformatics/btg405.
- Haferlach, T., Kohlmann, A., Wieczorek, L., Basso, G., Kronnie, G.T., Bene, M.C., De Vos, -J., Hernandez, J.M., Hofmann, W.K., Mills, K.I., et al. (2010). Clinical utility of microarray-based gene expression profiling in the diagnosis and subclassification of leukemia: report from the International Microarray Innovations in Leukemia Study Group. J Clin Oncol 28, 2529-2537. 10.1200/JCO.2009.23.4732.
- Heidel, F.H., Bullinger, L., Arreba-Tutusaus, P., Wang, Z., Gaebel, J., Hirt, C., Niederwieser, D., Lane, S.W., Dohner, K., Vasioukhin, V., et al. (2013). The cell fate determinant Llgll influences HSC fitness and prognosis in AML J Exp Med 210, 15-22. 10.1084/jem.20120596.
- Heidel, F.H., Bullinger, L., Feng, Z., Wang, Z., Neff, T.A., Stein, L., Kalaitzidis, D., Lane, S.W., and Armstrong, S.A. (2012). Genetic and pharmacologic inhibition of beta-catenin targets imatinib- resistant leukemia stem cells in CML. Cell Stem Cell 10, 412-424. 10.1016/j.stem.2012.02.017.
- Krivtsov, A.V., and Armstrong, S.A. (2007). MLL translocations, histone modifications and leukaemia stem-cell development. Nat Rev Cancer 7, 823-833. 10.1038/nrc2253.
- Krivtsov, A.V., Twomey, D., Feng, Z., Stubbs, M.C., Wang, Y., Faber, J., Levine, J.E., Wang, J., Hahn, W.C., Gilliland, D.G., et al. (2006). Transformation from committed progenitor to leukaemia stem cell initiated by MLL-AF9. Nature 442, 818-822. 10.1038/nature04980.
- Kuhn, M.W., Song, E., Feng, Z., Sinha, A., Chen, C.W., Deshpande, A.J., Cusan, M., Farnoud, N., Mupo, A., Grove, C., et al. (2016). Targeting Chromatin Regulators Inhibits Leukemogenic Gene Expression in NPM1 Mutant Leukemia. Cancer Discov 6, 1166-1181. 10.1158/2159-8290.CD-16- 0237.
- Love, M.I., Huber, W., and Anders, S. (2014). Moderated estimation of fold change and dispersion for RNA-seq data with DESeq2. Genome biology 15, 550. 10.1186/s13059-014-0550-8.
- Muchamuel, T., Basler, M., Aujay, M.A., Suzuki, E., Kalim, K.W., Lauer, C., Sylvain, C., Ring, E.R., Shields, J., Jiang, J., et al. (2009). A selective inhibitor of the immunoproteasome subunit LMP7 blocks cytokine production and attenuates progression of experimental arthritis. Nat Med 15, 781-787. 10.1038/nm.1978.
- Reich, M., Liefeld, T., Gould, J., Lerner, J., Tamayo, P., and Mesirov, J.P. (2006). GenePattern 2.0. Nat Genet 38, 500-501. 10.1038/ng0506-500.
- Schnoder, T.M., Arreba-Tutusaus, P., Griehl, I., Bullinger, L., Buschbeck, M., Lane, S.W., Dohner, K., Plass, C., Lipka, D.B., Heidel, F.H., and Fischer, T. (2015). Epo-induced erythroid maturation is dependent on Plcgammal signaling. Cell Death Differ 22, 974-985. 10.1038/cdd.2014.186.
- Stavropoulou, V., Almosailleakh, M., Royo, H., Spetz, J.F., Juge, S., Brault, L., Kopp, P., Iacovino, M., Kyba, M., Tzankov, A., et al. (2018). A Novel Inducible Mouse Model of MLL-ENL-driven Mixed- lineage Acute Leukemia. Hemasphere 2, e51. 10.1097/HS9.0000000000000051.
- Stein, E.M., Aldoss, I., DiPersio, J.F., Stone, R.M., Arellano, M.L., Rosen, G., Meyers, M.L., Huang, Y., Smith, S., Bagley, R.G., et al. (2021). Safety and Efficacy of Menin Inhibition in Patients (Pts) with MLL-Rearranged and NPM1 Mutant Acute Leukemia: A Phase (Ph) 1, First-in-Human Study of SNDX-5613 (AUGMENT 101). Blood Volume 138 (Supplement 1).
- Stein, E.M., Garcia-Manero, G., Rizzieri, D.A., Tibes, R., Berdeja, J.G., Savona, M.R., Jongen- Lavrenic, M., Altman, J.K., Thomson, B., Blakemore, S.J., et al. (2018). The DOT1L inhibitor pinometostat reduces H3K79 methylation and has modest clinical activity in adult acute leukemia. Blood 131, 2661-2669. 10.1182/blood-2017-12-818948.
- Uckelmann, H.J., Kim, S.M., Wong, E.M., Hatton, C., Giovinazzo, H., Gadrey, J.Y., Krivtsov, A.V., Rucker, F.G., Dohner, K., McGeehan, G.M., et al. (2020). Therapeutic targeting of preleukemia cells in a mouse model of NPM1 mutant acute myeloid leukemia. Science 367, 586-590. 10.1126/science.aax5863.
- Wang, B., Wang, M., Zhang, W., Xiao, T., Chen, C.H., Wu, A., Wu, F., Traugh, N., Wang, X., Li, Z., et al. (2019). Integrative analysis of pooled CRISPR genetic screens using MAGeCKFlute. Nat Protoc 14, 756-780. 10.1038/s41596-018-0113-7.
- Wolf-Levy, H., Javitt, A., Eisenberg-Lerner, A., Kacen, A., Ulman, A., Sheban, D., Dassa, B., Fishbain- Yoskovitz, V., Carmona-Rivera, C., Kramer, M.P., et al. (2018). Revealing the cellular degradome by mass spectrometry analysis of proteasome-cleaved peptides. Nat Biotechnol. 10.1038/nbt.4279.
- Wouters, B.J., Lowenberg, B., Erpelinck-Verschueren, C.A., van Putten, W.L., Valk, P.J., and Delwel, R. (2009). Double CEBPA mutations, but not single CEBPA mutations, define a subgroup of acute myeloid leukemia with a distinctive gene expression profile that is uniquely associated with a favorable outcome. Blood 113, 3088-3091. 10.1182/blood-2008-09-179895.
- Xu, H., Xiao, T., Chen, C.H., Li, W., Meyer, C.A., Wu, Q., Wu, D., Cong, L., Zhang, F., Liu, J.S., et al. (2015). Sequence determinants of improved CRISPR sgRNA design. Genome Res 25, 1147-1157. 10.1101/gr.191452.115.

## Claims

1. Combination of at least one menin inhibitor with at least one immunoproteasome inhibitor for use as medicament.

2. Combination of at least one menin inhibitor with at least one immunoproteasome inhibitor for use in treatment of leukemia.

3. Combination for use according to claim 1 or 2, wherein the at least one menin inhibitor is a compound selected from the group consisting of MI-503, SNDX-5613, KO-539, MI-3454, mixtures of two or more of these menin inhibitors.

4. Combination for use according to claim 3, wherein the at least one menin inhibitor comprises at least MI-503.

5. Combination for use according to any one of claims 1 to 4, wherein the at least one immunoproteasome inhibitor is a compound selected from the group consisting of Bortezomib, Carfilzomib, PR-957 (ONX-0914), M3258 and mixtures of two or more of these immunoproteasome inhibitors.

6. Combination for use according to claim 5, wherein the at least one immunoproteasome inhibitor comprises at least PR-957.

7. Combination for use according to any one of claims 1 to 6, wherein the at least one menin inhibitor and the at least one immunoproteasome inhibitor are present in a molar ratio at least one menin inhibitor : at least one immunoproteasome inhibitor in the range of from 1:100 to 100:1, preferably in a molar ratio in the range of from 1:10 to 10:1.

8. Combination for use according to any one of claims 1 to 7, wherein the at least menin inhibitor and the at least one immunoproteasome inhibitor are administered simultaneously consecutively or staggered, wherein in a staggered administration, preferably the administration of the at least one menin inhibitor starts prior to administration of the at least one immunoproteasome inhibitor.

9. Combination for use according to claim 8, wherein the at least one menin inhibitor is administered, preferably daily, for 1 to 14 consecutive days prior to start of the administration of the at least one immunoproteasome inhibitor.

10. Combination for use according to claim 8 or 9, wherein in simultaneous administration or in staggered administration after start of the administration of the at least one immunoproteasome inhibitor, the at least one menin inhibitor and the at least one immunoproteasome inhibitor are administrated, preferably both daily, together for a period of time of at least two days.

11. Combination for use according to any one of claims 1 to 10, wherein the leukemia is selected from the group of acute myeloid leukemias (AML), and is preferably an AML associated with rearrangement(s) in the Mixed-Lineage-Leukemia gene 1 (MLL1-r-AML) or an AML associated with NPM1 mutation(s).

12. Pharmaceutical preparation comprising at least one menin inhibitor and at least one immunoproteasome inhibitor, optionally one or more pharmaceutically acceptable carrier(s) and optionally one or more pharmaceutically acceptable adjuvant(s).

13. Pharmaceutical preparation comprising at least one menin inhibitor and at least one immunoproteasome inhibitor according to claim 12, wherein the at least one menin inhibitor and at least one immunoproteasome inhibitor are present in the same dosage unit or are present in separated dosage units.

14. Pharmaceutical preparation comprising at least one menin inhibitor and at least one immunoproteasome inhibitor according to claim 12 or 13, comprising one or more dosage units, wherein the at least one menin inhibitor and at least one immunoproteasome inhibitor are present together in each dosage unit, optionally combined with one or more dosage units comprising the at least one menin inhibitor as the sole active ingredient.

15. Pharmaceutical preparation comprising at least one menin inhibitor and at least one immunoproteasome inhibitor according to claim 12 or 13, comprising one or more dosage units comprising the at least one menin inhibitor as the sole active ingredient and one or more dosage units comprising the at least one immunoproteasome inhibitor as the sole active ingredient, wherein optionally, the sum of dosage units comprising the at least one menin inhibitor as the sole active ingredient is larger than the sum of dosage units comprising the at least one immunoproteasome inhibitor as the sole active ingredient.
